(19) Europäisches Patentamt
European Patent Office
Office européen des brevets

(11) Veröffentlichungsnummer: **0 098 243**
**B1**

(12) # EUROPÄISCHE PATENTSCHRIFT

(45) Veröffentlichungstag der Patentschrift: **07.01.87**

(51) Int. Cl.⁴: **C 07 D 249/08**, C 07 D 233/64, C 07 D 401/06, C 07 D 403/06, C 07 D 405/12, C 07 D 409/12, A 01 N 43/653, A 01 N 43/50

(21) Anmeldenummer: **83810271.3**

(22) Anmeldetag: **20.06.83**

(54) **Neue Säure-Derivate, Verfahren zu ihrer Herstellung und ihre Verwendung zur Bekämpfung von Mikroorganismen.**

(30) Priorität: **25.06.82 CH 3922/82**

(43) Veröffentlichungstag der Anmeldung:
**11.01.84 Patentblatt 84/2**

(45) Bekanntmachung des Hinweises auf die Patenterteilung:
**07.01.87 Patentblatt 87/2**

(84) Benannte Vertragsstaaten:
**AT BE CH DE FR GB IT LI NL SE**

(56) Entgegenhaltungen:
**EP - A - 0 013 786**
**EP - A - 0 041 673**
**EP - A - 0 056 860**
**EP - B - 0 000 752**
**DE - A - 3 129 193**

**CHEMICAL ABSTRACTS, Vol. 92, Nr. 21, 26. Mai 1980, Columbus, Ohio, USA T. MAIER "Beta-Azolyl-alpha,alpha-dicarbonyl compounds" Seite 648, Spalte 1, Zusammenfassung-Nr. 181 098f**

(73) Patentinhaber: **CIBA-GEIGY AG, Klybeckstrasse 141, CH-4002 Basel (CH)**

(72) Erfinder: **Kunz, Walter, Dr., Im Goldbrunnen 55, CH-4104 Oberwil (CH)**
Erfinder: **Eckhardt, Wolfgang, Dr., Breslauerstrasse 16, D-7850 Lörrach (DE)**

**EP 0 098 243 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Mandelsäurederivate der nachstehenden Formel I sowie deren pflanzenverträgliche Säureadditionssalze, und Metallkomplexe. Sie betrifft ferner die Herstellung dieser Verbindungen sowie agrochemische Mittel, die als Wirkstoff mindestens eine der Verbindungen der Formel I enthalten; sie betrifft auch die Herstellung der Mittel und ein Verfahren zur Bekämpfung oder präventiven Verhütung eines Befalls von Pflanzen durch Mikroorganismen.

Die erfindungsgemässen Verbindungen gehorchen der Formel I

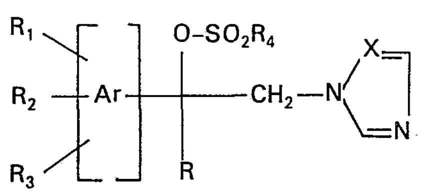

(I),

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$–$C_3$-Alkyl, $C_1$–$C_3$-Alkoxy oder $C_1$–$C_3$-Haloalkyl stehen;

R eine der Gruppen $-COOR_5$, $-COSR_6$,

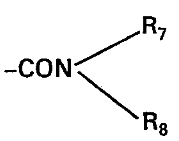

oder -CN darstellt;

$R_5$ ein unsubstituiertes oder durch Halogen substituiertes

$C_2$–$C_{10}$-Alkenyl; ein unsubstituiertes oder durch Halogen substituiertes $C_2$–$C_{10}$-Alkinyl; oder eine $C_3$–$C_8$-Cycloalkylgruppe oder eine unsubstituierte oder durch Halogen, $C_1$–$C_4$-Alkyl, $C_1$–$C_4$-Alkoxy, -CN oder $-CF_3$ substituierte Phenylgruppe bedeutet; oder aber eine $C_1$–$C_{12}$-Alkylkette darstellt, die ab $C_2$-Alkyl durch Sauerstoff oder Schwefel unterbrochen und die unsubstituiert oder durch eines der folgenden Atome oder Gruppen substituiert sein kann: Halogen, Phenyl, -COOAlkyl($C_1$–$C_4$), -COAlkyl($C_1$–$C_4$), -COPhenyl, einen ungesättigten oder gesättigten 5- oder 6-gliedrigen Ring mit Sauerstoff oder Schwefel als Heteroatom, wobei jeder Phenylrest unsubstituiert oder ein- oder mehrfach durch gleiche oder verschiedene Halogenatome substituiert ist;

$R_6$ $C_1$–$C_{10}$-Alkyl oder eine unsubstituierte oder durch Halogen, $C_1$–$C_4$-Alkyl, $C_1$–$C_4$-Alkoxy, -CN oder $-CF_3$ substituierte Phenyl- oder Benzylgruppe bedeutet;

$R_7$ und $R_8$ unabhängig voneinander Wasserstoff, $C_1$–$C_6$-Alkyl, $C_3$–$C_7$-Cycloalkyl oder eine Phenyl- oder Benzylgruppe darstellen, bei denen jeweils der aromatische Ring unsubstituiert oder durch Halogen, $C_1$–$C_4$-Alkyl, $C_1$–$C_4$-Alkoxy, -CN oder $-CF_3$ substituiert ist, oder $R_7$ und $R_8$ zusammen einen 5- oder 6-gliedrigen gesättigten oder ungesättigten heterocyclischen Ring bilden, der noch 1 oder 2 weitere N-Atome enthalten kann;

$R_4$ gegebenenfalls durch Halogen oder $C_1$–$C_4$-Alkoxy substituiertes $C_1$–$C_{12}$-Alkyl, gegebenenfalls durch Halogen, $C_1$–$C_4$-Alkyl, $C_1$–$C_4$-Alkoxy, $C_1$–$C_4$-Haloalkyl oder Cyano substituiertes Phenyl oder ebenso gegebenenfalls substituiertes Benzyl bedeutet oder für die Gruppe $-N(R_9)(R_{10})$ steht; wobei

$R_9$ $C_1$–$C_6$-Alkyl bedeutet; und

$R_{10}$ für Wasserstoff, $C_1$–$C_4$-Alkyl, $C_1$–$C_4$-Haloalkylsulfenyl, $-SC_6H_5$ oder $-SC(CH_3)_2CN$ steht;

wobei Säureadditionssalze sowie Metallkomplexe der Formel I mit eingeschlossen sind.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl, sowie ihre Isomeren, wie z.B. Isopropyl, Isobutyl, tert.-Butyl, Isopentyl usw. Alkenyl steht z.B. für Vinyl, Propenyl-(1), Allyl, Butenyl-(1), Butenyl-(2), Butenyl-(3) usw., sowie Ketten mit mehreren Doppelbindungen. Alkinyl bedeutet z.B. Propinyl-(1), Propargyl, Butinyl-(1), Butinyl-(2) usw., bevorzugt Propargyl, durch Halogen substituiertes Alkyl steht insbesondere für einen ein- bis perhalogenierten Alkylsubstituenten, wie z.B. $CHCl_2$, $CH_2Cl$, $CCl_3$, $CF_3$, $CH_2CH_2Cl$, usw. Unter Halogen soll hier und im folgenden Fluor, Chlor, Brom oder Jod, vorzugsweise Chlor, Brom oder Fluor verstanden werden. Cycloalkyl steht z.B. für Cyclopropyl, Cyclobutyl, Cyclopentyl, Cyclohexyl oder Cycloheptyl, bevorzugt für Cyclopropyl und Cyclohexyl. Haloalkenyl steht für eine ein- oder mehrfach durch Halogen substituierte Alkenylgruppe, wobei als Halogen Chlor und Brom, insbesondere Chlor bevorzugt sind. Furyl steht bevorzugt für 2-Furyl, Tetrahydrofuryl bevorzugt für 2-Tetrahydrofuryl, Pyridyl bedeutet vor allem 3- oder 4-Pyridyl, Naphthyl steht für α- oder β-Naphthyl, insbesondere für α-Naphthyl. Beispiele heterocyclischer 5- bzw. 6-Ringe mit bis zu 3 N-Atomen sind Pyrazol, Imidazol, 1,2,4-Triazol und 1,3,4-Triazol, Pyridin, Pyrazin, Pyrimidin, Pyridazin, 1,3,5-Triazin und 1,2,4-Triazin.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus

dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Bezoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan und Zinn sind bevorzugt.

Die Verbindungen der Formel I sind bei Raumtemperatur stabile Öle, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I (X bedeutet N) bevorzugt. Die erfindungsgemässen Wirkstoffe der Formel I zeichnen sich durch eine sehr gute Pflanzenverträglichkeit aus. Die Entwicklung der Pflanzen wird in keinem Stadium behindert oder verzögert.

Eine wichtige und bevorzugte Untergruppe betrifft Verbindungen der Formel I*

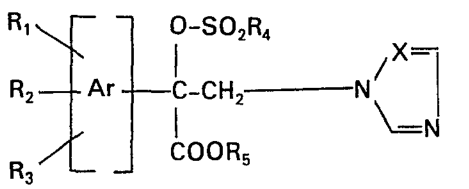

(I*),

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen,

$C_1$-$C_3$-Alkyl, $C_1$-$C_3$-Alkoxy oder $C_1$-$C_3$-Haloalkyl stehen;

$R_5$ $C_1$-$C_4$-Alkyl, Phenyl, ein durch Nitro, Halogen und/oder Methyl ein- oder mehrfach substituiertes Phenyl oder Benzyl bedeutet;

$R_4$ für $C_1$-$C_6$-Alkyl, gegebenenfalls durch Fluor, Chlor, Brom, $CF_3$, Methyl, Methoxy oder Cyano substituiertes Phenyl oder ebenso gegebenenfalls substituiertes Benzyl steht oder die Gruppe $-N(R_9)(R_{10})$ repräsentiert; wobei

$R_9$ $C_1$-$C_3$-Alkyl bedeutet; und

$R_{10}$ für Wasserstoff, $C_1$-$C_3$-Alkyl, $-SCCl_3$, $-SCCl_2F$, $-SCCl_2CHCl_2$, $-SC_6H_5$ oder $-SC(CH_3)_2CN$ steht;

unter Einschluss der pflanzenverträglichen Säureadditionssalze sowie Metallkomplexe.

Eine andere bevorzugte Untergruppe bilden Verbindungen der Formel I, worin X für das Brückenglied -CH= oder -N= steht; Ar eine Phenylgruppe bedeutet; $R_1$ in ortho-Position für Wasserstoff, Nitro, Halogen, $C_1$-$C_3$-Alkyl, $C_1$-$C_3$-Alkoxy oder $C_1$-$C_3$-Haloalkyl steht; $R_2$ in para-Position für Wasserstoff, Nitro, Halogen, $C_1$-$C_3$-Alkyl, $C_1$-$C_3$-Alkoxy oder $C_1$-$C_3$-Haloalkyl steht; $R_3$ Wasserstoff, Methyl oder Halogen bedeutet; R eine der Gruppen $-COOR_5$, $-COSR_6$,

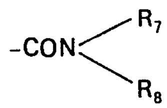

oder -CN darstellt; $R_5$ $C_1$-$C_4$-Alkyl, Phenyl, ein durch Nitro, Halogen und/oder Methyl ein- oder mehrfach substituiertes Phenyl oder ebenso substituiertes Benzyl bedeutet; $R_6$ $C_1$-$C_{10}$-Alkyl oder eine unsubstituierte oder durch Halogen, $C_1$-$C_4$-Alkyl, $C_1$-$C_4$-Alkoxy, -CN oder $-CF_3$ substituierte Phenyl- oder Benzylgruppe bedeutet, $R_7$ und $R_8$ unabhängig voneinander für Wasserstoff, $C_1$-$C_3$-Alkyl, $C_3$-$C_7$-Cycloalkyl, Phenyl oder Benzyl stehen; und $R_4$ für $C_1$-$C_4$-Alkyl, gegebenenfalls durch Fluor, Chlor, Brom, $CF_3$, Methyl oder Methoxy substituiertes Phenyl oder gegebenenfalls ebenso substituiertes Benzyl steht oder die Gruppe $-N(R_9)(R_{10})$ repräsentiert; wobei $R_9$ für $C_1$-$C_3$-Alkyl steht; und $R_{10}$ Wasserstoff, $C_1$-$C_3$-Alkyl, $-SCCl_3$, $-SCCl_2F$, $-SCCl_2CHCl_2$, $-SC_6H_5$ oder $-SC(CH_3)_2CN$ bedeutet; wobei Säureadditionssalze sowie Metallkomplexe der Formel I mit eingeschlossen sind. Diese Untergruppe soll hier und im folgenden mit dem Symbol I** bezeichnet werden.

Eine weitere, besonders bevorzugte Untergruppe bilden Verbindungen der Formel I, worin X für das Brückenglied -N= steht;

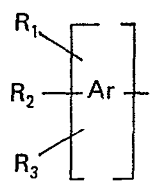

eine in ortho- und/oder para-Position durch Nitro, Fluor, Chlor, Brom, Methyl, Methoxy und/oder $CF_3$ substituierte Phenylgruppe bedeutet; R für die Gruppe $-COOR_5$ steht; $R_5$ $C_1$-$C_4$-Alkyl, Phenyl, ein durch Nitro, Chlor, Brom, Fluor und/oder Methyl substituiertes Phenyl oder ebenso substituiertes Benzyl bedeutet; und $R_4$ für Methyl, Ethyl, Phenyl, p-Tolyl, p-Methylbenzyl, $-NH(C_1$-$C_4$-Alkyl) oder $-N(R_9)(R_{10})$ steht; wobei $R_9$ $C_1$-$C_2$-Alkyl und $R_{10}$ Methyl, $-SCCl_3$, $-SCCl_2F$, $-SCCl_2CHCl_2$, $-SC_6H_5$ oder $-SC(CH_3)_2CN$ bedeuten; wobei Säureadditionssalze sowie Metallkomplexe der Formel I mit eingeschlossen sind. Diese Untergruppe soll hier und im folgenden mit dem Symbol I*** bezeichnet werden.

Folgende Einzelsubstanzen sind als Mikrobizide besonders bevorzugt:

2-(1H-1,2,4-Triazolylmethyl-1′-yl)-2-methylsulfo-

nyloxy-2-chlor-4-bromphenylessigsäureethylester;
2-(1H-1,2,4-Triazolylmethyl-1'-yl)-2-methylsulfonyloxy-2,4-dichlorphenylessigsäureethylester;
2-(1H-1,2,4-Triazolylmethyl-1'-yl)-2-(N-methyl-N-fluordichlormethansulfenyl-sulfamoyloxy)-2-chlor-4-bromphenylessigsäureethylester;
2-(1H-1,2,4-Triazolylmethyl-1'-yl)-2-(N-ethylsulfamoyloxy)-2,4-dichlorphenylessigsäuremethylester;
2-(1H-1,2,4-Triazolylmethyl-1'-yl)-2-(N-methylsulfamoyloxy)-2-chlor-4-bromphenylessigsäureethylester;
2-(1H-1,2,4-Triazolylmethyl-1'-yl)-2-(N,N-dimethylsulfamoyloxy)-2-chlor-4-bromphenylessigsäureethylester;
2-(1H-1,2,4-Triazolylmethyl-1'-yl)-2-methylsulfonyloxy-4-chlorphenylessigsäureethylester;
2-(1H-1,2,4-Triazolylmethyl-1'-yl)-2-(N-methylsulfonyloxy)-4-chlorphenylessigsäureethylester;
2-(1H-1,2,4-Triazolylmethyl-1'-yl)-2-(N-methyl-N-fluordichlormethansulfenyl-sulfamoyloxy)-4-chlorphenylessigsäureethylester;
2-(1H-1,2,4-Triazolylmethyl-1'-yl)-2-methylsulfonyloxy-2-chlor-4-bromphenylessigsäureisopropylester;
2-(1H-1,2,4-Triazolylmethyl-1'-yl)-2-ethylsulfonyloxy-2-chlor-4-bromphenylessigsäureethylester;
2-(1H-1,2,4-Triazolylmethyl-1'-yl)-2-chlormethylsulfonyloxy-2-chlor-4-bromphenylessigsäureethylester;
2-(1H-1,2,4-Triazolylmethyl-1'-yl)-2-paratosyloxy-2-chlor-4-bromphenylessigsäureethylester;
2-(1H-1,2,4-Triazolylmethyl-1'-yl)-2-(N-trichlormethansulfenylsulfamoyloxy)-2-chlor-4-bromphenylessigsäureethylester;
2-(1H-1,2,4-Triazolylmethyl-1'-yl)-2-methylsulfonyloxy-2,4-dichlorphenylessigsäuremethylester;
2-(1H-1,2,4-Triazolylmethyl-1'-yl)-2-methylsulfonyloxy-2,4-dichlorphenylessigsäure-n-butylester.

Ganz besonders bevorzugt sind die Verbindungen Nr. 1.8, 1.34, 1.60 u. 1.62.

Die Verbindungen der Formel I werden erfindungsgemäss dadurch hergestellt, dass man einen zugrundeliegenden Alkohol der Formel II

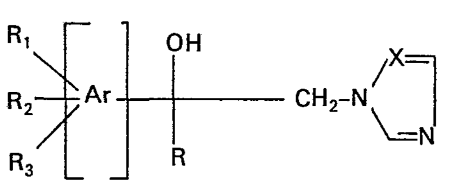
(II)

entweder direkt in Gegenwart einer organischen oder anorganischen Base mit einem Sulfonylhalogenid der Formel XVI

$$HalSO_2R_4 \qquad (XVI)$$

an der OH-Gruppe sulfonyliert oder vorteilhafterweise den Alkohol II vor der Sulfonylierung mit XVI zuerst durch Reaktion mit einem Alkalihydrid, wie NaH, KH, usw. oder einer alkaliorganischen Verbindung, wie z.B. Butyllithium in ein Alkalialkoholat der Formel III

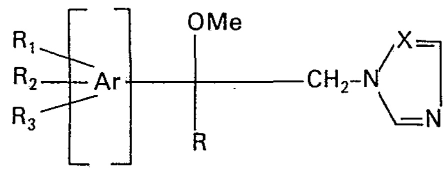
(III)

überführt und, wenn erwünscht, die verfahrensgemäss erhältliche Verbindung der Formel I in eine andere Verbindung der Formel I umwandelt und/oder eine verfahrensgemäss erhältliche freie Verbindung in ein Säureadditionssalz umwandelt, ein verfahrensgemäss erhältliches Säureadditionssalz in die freie Verbindung oder ein anderes Säureadditionssalz, oder eine verfahrensgemäss erhältliche freie Verbindung bzw. ein verfahrensgemäss erhältliches Salz in einen Metallkomplex überführt. Hierbei haben die Substituenten R, $R_1$, $R_2$, $R_3$, $R_4$, X und Ar in den Formeln II, III und XVI die unter Formel I angegebenen Bedeutungen, und Hal in Formel XVI steht für ein Halogenatom, insbesondere für Chlor oder Brom und Me repräsentiert ein Alkalimetallatom wie Lithium, Kalium oder Natrium.

Die Reaktion kann in Ab- oder vorzugsweise Anwesenheit eines reaktionsinerten Lösungs- oder Verdünnungsmittels bei Temperaturen von −20°C bis +150°C durchgeführt werden, wobei die direkte Sulfonylierung von II mit XVI vorzugsweise bei 0° bis +40°C und die indirekte Sulfonylierung von III mit XVI vorzugsweise bei 0° bis +25°C erfolgt.

Geeignete reaktionsinerte Lösungs- und Verdünnungsmittel sind beispielsweise aliphatische und aromatische Kohlenwasserstoffe wie Benzol, Toluol, Xylole, Petrolether, Cyclohexan usw. Ether und etherartige Verbindungen wie Dialkylether (Diethylether, Diisopropylether, tert.-Butylmethylether usw.), Anisol, Dioxan, Tetrahydrofuran, Furan usw.; N,N-dialkylierte Amide wie Dimethylformamid; Dimethylsulfoxid; im Falle der Umsetzung von II mit XVI auch zusätzlich halogenierte aromatische und aliphatische Kohlenwasserstoffe wie Chlorbenzol, Methylenchlorid, Ethylenchlorid, Chloroform, Tetrachlorkohlenstoff, Tetrachlorethylen, usw. – und in beiden Fällen auch Gemische derartiger Lösungsmittel untereinander. In einer besonders bevorzugten Ausführungsform der Sulfonylierung von II mit XVI arbeitet man in einem Gemisch aus Methylenchlorid und Triethylamin in Gegenwart einer katalytischen Menge von 4-Dimethylaminopyridin.

In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid. Ferner kann das Arbeiten unter erhöhtem Druck und/oder absoluten

Lösungsmitteln sich günstig auf die Ausbeute auswirken.

Geeignete anorganische Basen sind beispielsweise Oxide, Hydride, Hydroxide, Carbonate, Carbonsäuresalze und Alkoholate der Erdalkali-, bevorzugt der Alkalimetalle, insbesondere die des Natriums und Kaliums (z.B. NaH, NaOH, KOH, $Na_2CO_3$, $K_2CO_3$, $CaCO_3$, $CH_3COONa$, $C_2H_5COOK$, $C_2H_5ONa$, $CH_3ONa$, usw.), bevorzugt die Alkalihydride wie NaH. Als organische Basen eignen sich Trialkylamine wie z.B. Triethylamin oder andere tertiäre Amine wie beispielsweise Triethylendiamin, Piperidin, Pyridin, 4-Dimethylaminopyridin, 4-Pyrrolidylpyridin usw.

Bei den erfindungsgemässen Herstellungsverfahren für die Endprodukte sowie bei der Herstellung der Ausgangsstufen können die anfallenden Zwischen- und End-Produkte aus dem Reaktionsmedium isoliert und, falls gewünscht, auf eine der allgemein üblichen Methoden gereinigt werden, z.B. durch Extraktion, Kristallisation, Chromatographie, Destillation usw. Man kann die Herstellung der Verbindungen der Formel I jedoch im allgemeinen auch in einer sogenannten Eintopfreaktion durchführen. Besonders vorteilhafte Reaktionsvarianten zur Herstellung der Verbindungen der Formel I sowie zur Herstellung der Zwischenprodukte, insbesondere derjenigen der Formel II, werden anschliessend in einem Reaktionsschema skizziert und darauffolgend im einzelnen beschrieben.

In den Formeln Ia, Ib, II, III, IV, V, VI, VII, VIII, IX, X, XI, XII, XIII, XIV, XV und XVI haben die Substituenten Ar, X, R, $R_1$, $R_2$, $R_3$, $R_4$, $R_5$, $R_6$, $R_7$ und $R_8$ die unter Formel I angegebene Bedeutungen.

Q steht in Formel XV entweder für eine der üblichen Abgangsgruppen z.B. Halogen, insbesondere Chlor, Brom oder Jod; für eine Sulfonyloxygruppe, insbesondere Benzolsulfonyloxy, Paratosyloxy oder Niederalkylsulfonyloxy, bevorzugt Mesyloxy; oder für eine Acyloxygruppe wie Trifluoracetyloxy. Q steht auch für eine Hydroxygruppe oder gemäss «Synthesis» 1979, p. 561–569, für den Rest

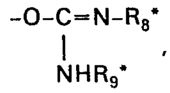

,

worin $R_8^*$ und $R_9^*$ Organylreste, insbesondere Niederalkyl oder gegebenenfalls substituierte Phenylreste repräsentieren. M steht für Wasserstoff oder ein Metallatom, insbesondere für ein Alkalimetallatom, vorzugsweise für Natrium oder Kalium. Hal steht für Halogen, bevorzugt für Chlor oder Brom. Me bedeutet ein Alkalimetallatom, insbesondere Lithium, Natrium oder Kalium.

Das Symbol α steht stellvertretend für die Gruppierung

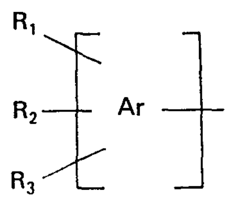

worin die Substituenten $R_1$, $R_2$, $R_3$ und Ar wie unter Formel I definiert sind.

Az repräsentiert die nachfolgende Azolylgruppe

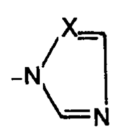

worin X für -CH= oder -N= steht.

Reaktionsschema

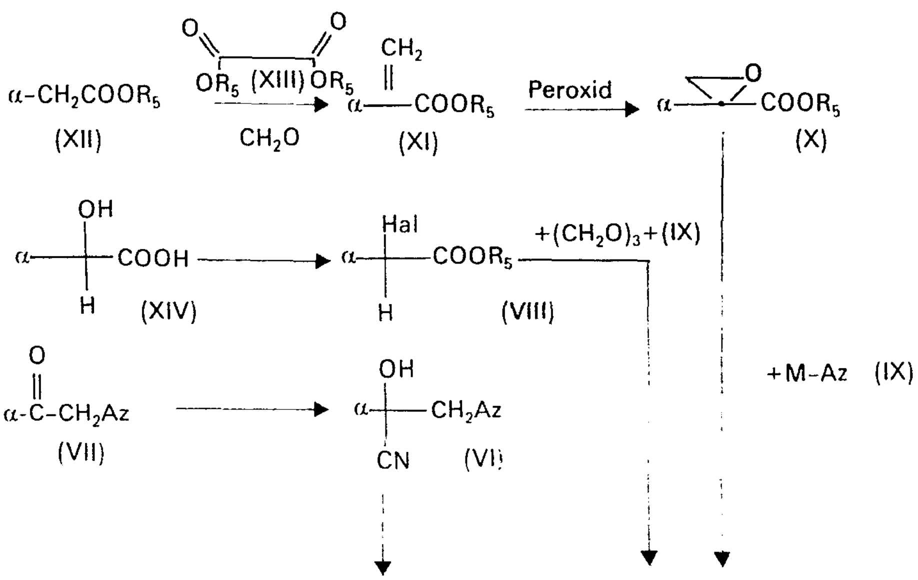


(XII) (XI) (X) (XIV) (VIII) (VII) (VI)

Reaktionsschema (Fortsetzung)

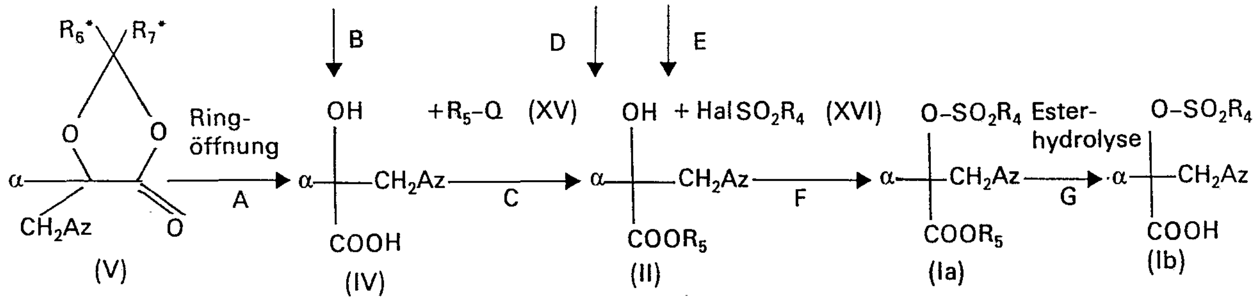


Zur Herstellung der Zwischenprodukte sowie der Endprodukte der Formel I kann man im einzelnen wie folgt vorgehen:

i) Freie α-Hydroxycarbonsäuren (= Mandelsäuren) der Formel IV werden dadurch hergestellt, dass man wahlweise, entweder nach Gleichung A ein Dioxolanon der Formel V oder nach Gleichung B ein Cyanhydrin der Formel VI basisch oder sauer hydrolysiert.

Die Hydrolysereaktionen A und B werden mit Säuren oder Basen vorteilhafterweise in wässrigen und/oder alkoholischen Lösungen, also in polaren Lösungsmitteln, durchgeführt. Die Reaktionen können auch in zweiphasigen Medien durchgeführt werden. Hierbei ist die Zugabe eines üblichen Phasentransfer-Katalysators vorteilhaft. Es kommen anorganische und organische Säuren in Frage, beispielsweise Mineralsäuren wie Halogenwasserstoffsäuren, Schwefelsäure, Phosphorsäure oder Sulfonsäuren (p-Toluolsulfonsäure, Methansulfonsäure). Als Basen eignen sich organische und anorganische Basen, beispielsweise Oxide, Hydride, Hydroxide, Carbonate, Carbonsäuresalze und Alkoholate der Erdalkali- und der Alkali-Metalle, insbesondere der des Natriums und Kaliums.

Die Reaktionstemperaturen liegen bei der Ringöffnungsreaktion A im allgemeinen zwischen 0° und +140°C, bevorzugt zwischen +30° und +80°C und bei der Hydrolyse des Cyanhydrins III zwischen +60° und +140°C, bevorzugt +80° und +120°C oder jeweils am Siedepunkt des Lösungsmittels oder Lösungsmittelgemisches.

Ausgangsverbindungen der Formel V sind grösstenteils aus der EP-OS Nr. 44 276 bekannt. Die noch neuen Verbindungen werden analog den dort angegebenen Methoden hergestellt.

Die Mandelonitrile VI (Variante B) lassen sich auf übliche Art aus Aryl-azolylmethylketonen der Formel VII

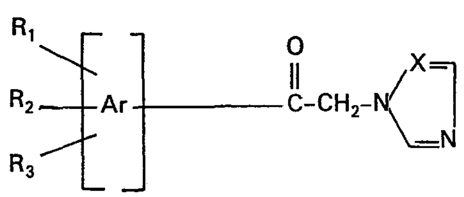 (VII)

nach Art einer Cyanhydrin-Synthese durch Reaktion mit HCN oder einem Alkalicyanid, wie z.B. KCN oder NaCN, bei 0° bis 100°C, vorteilhafterweise in Anwesenheit einer Spur einer Base (vorzugsweise $NH_4OH$ oder gasförmigem Ammoniak) oder über das korrespondierende $NaHSO_3$-Addukt VII herstellen [Org. Syntheses Coll. Vol. I, p. 336, oder FR-PS 2 292 706; oder Houben-Weyl «Methoden der organischen Chemie», Band 6/3, p. 412].

Mandelonitrile VI lassen sich auch gemäss J. Org. Chem. 39, p. 914 (1974) durch Reaktion von VII mit Trimethylsilylcyanid in Gegenwart katalytischer Mengen $ZnJ_2$ und anschliessende Hydrolyse des Additionsprodukts herstellen.

Sie lassen sich auch durch Reaktion eines Ketons VII mit einem an sich bekannten Diniederalkylcyanhydrin der Formel

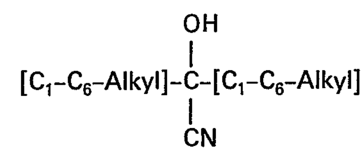

(Alkyl ist besonders Methyl, Ethyl, Propyl) bevorzugt in einem inerten Lösungsmittel oder ohne Lösungsmittel bei 50–150°C gewinnen.

Die Hydrolyse der Nitrile VI zu Mandelsäurederivaten der Formel IV kann analog zu bekannten Methoden z.B. mit konz. Chlorwasserstoffsäure erfolgen [Houben-Weyl «Methoden der organischen Chemie, Band VIII, p. 427 ff. (1952)].

Die als Zwischenprodukte dienenden Ketone der Formel VII sind aus der DE-OS 2 431 407 bzw. der GB-PS 1 464 224 zum Teil bekannt geworden. Derartige Ketone lassen sich auch durch Hydrolyse aus entsprechenden Ketalen herstellen, z.B. aus solchen, die in einer der folgenden Publikationen genannt sind: DE-OS 2 610 022; 2 602 770; 2 930 029; 2 930 196; 2 940 133.

Noch nicht beschriebene Ketone der Formel VII lassen sich nach einer der vorstehend publizierten Methoden erhalten.

ii) Mandelsäureester der Formel II lassen sich gemäss Gleichung C auf übliche Art durch Veresterung des entsprechenden Mandelsäure-Derivats IV (auch in Form seines Alkalimetallsalzes) mit $R_5$-Q (XV) bei −20° bis +140°C herstellen. Für diese Reaktion sind aprotische Lösungsmittel bevorzugt. Die direkte Veresterung wird vorteilhaft mit überschüssigem Alkohol $R_5$-OH bei 0° bis 80°C in Gegenwart von Mineralsäuren oder bevorzugt von Lewis-Säuren wie Bortrifluorid-Etherat durchgeführt.

Verbindungen der Formel II lassen sich auch gemäss Gleichung D mit Paraformaldehyd bei 20° bis 140°C, vorzugsweise 40° bis 80°C, und a) mit dem gewünschten Azo der Formel IX (Imidazol oder Triazol) in Gegenwart einer Base (z.B. NaOH) oder b) mit einem Alkalisalz des Azols der Formel IX in wasserfreien Lösungsmitteln (z.B. Dimethylsulfoxid) herstellen. Die α-Halogenessigester VIII sind durch übliche Veresterung aus den zugrundeliegenden, an sich bekannten Säuren XIV zugänglich.

Ester der Formel II lassen sich auch gemäss Gleichung E aus Oxiranen der Formel X mit einem Azol IX (M = H oder Alkalimetall) in einem inerten, bevorzugt polaren Lösungsmittel (DMF, Acetonitril, DMSO und anderen auch in Gemischen mit Kohlenwasserstoffen) bei 20° bis 100°C herstellen. Dabei können anorganische oder organische Basen zugesetzt werden [vgl. auch EP-OS 15 756].

Wie im Reaktionsschema skizziert, sind Oxirane der Formel X durch übliche Epoxidierung (z.B. $H_2O_2$/wss.NaOH, Peressigsäure u.a.) aus entsprechenden Alkenylverbindungen der Formel XI herstellbar. Verbindungen der Formel XI werden aus Arylessigsäureestern der Formel XII durch Reaktion mit Oxalsäureestern der Formel XIII und Formaldehyd in Gegenwart einer Base hergestellt [vgl. Helvetica Chimica Acta 30, p. 1349 (1947) und DE-OS 2 653 189].

Ester der Formel II lassen sich auch aus Säuren der Formel IV und Dimethylformamidacetal (vorzugsweise im Überschuss), dessen Acetalkomponente den alkoholischen Teil des Esters bilden soll, in Lösungsmitteln (z.B. ein gleichartiger wasserfreier Alkohol oder aber ein Ether) bei 0° bis 160°C herstellen [Angew. Chemie 75, p. 296 (1963) und Helv. Chim. Acta 48, 1747 (1965)].

iii) Verbindungen der Formel I lassen sich auf übliche Art in andere Verbindungen der Formel I umwandeln. So können Thiolether der Formel I aus den zugrundeliegenden freien Säuren der Formel I, z.B. durch Umsetzung mit entsprechenden Thioalkoholen in aprotischen Lösungsmitteln und vorzugsweise in Gegenwart schwacher Basen, hergestellt werden. Aus Estern oder Thioestern der Formel I können andererseits z.B. durch Umsetzung mit einem Überschuss eines entsprechenden Amins die Mandelsäureamide gewonnen werden. Sofern $R_7$ und $R_8$ zu einem 5- oder 6-gliedrigen Ring geschlossen sind, führt man solch einen Heterocyclus vorteilhaft durch Reaktion der zugrundeliegenden Säure mit 1,1′-Carbonyl-di-azol oder -azin bei 0° bis 150°C ein, vorzugsweise in Lösungsmitteln wie Ethern oder halogenierten Kohlenwasserstoffen. Darüberhinaus sind noch weitere Umwandlungen möglich.

iv) Die freie Hydroxylgruppe in den Verbindungen IV und II ist wie vorstehend beschrieben der Sulfonylierung mit Sulfonylhalogeniden der Formel XVI zugänglich.

Sämtliche vorstehend unter i), ii), iii) und iv) geschilderten Herstellungsvarianten sind Teil vorliegender Erfindung.

Die übrigen Ausgangsprodukte der Formeln III, IX, XII, XIII, XV und XVI sind bekannt oder werden nach an sich bekannten Methoden hergestellt. Die Verbindungen der Formel I besitzen nachbarständig zur aromatischen Gruppe Ar und zu R ein Asymmetriezentrum (*)

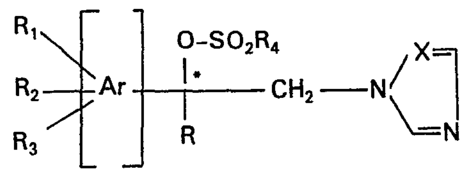 (I)

und können daher in zwei enantiomeren Formen vorliegen. Im allgemeinen entsteht bei der Herstellung dieser Substanzen ein Gemisch beider Enantiomeren; dieses lässt sich auf übliche Weise in die optischen Antipoden aufspalten. Optisch reine Antipoden erhält man beispielsweise nach Verfahrensvariante B, indem das Racemat der Säure IV mit einer optisch aktiven Base umgesetzt und das Salz durch fraktionierte Kristallisation trennt und nach Freisetzen der optisch reinen Säuren IV letztere wie unter Variante C zu den Estern II umgesetzt werden. Sulfonylierung dieser Ester liefert optisch reine Verbindungen der Formel Ia, welche gegebenenfalls in Folgereaktionen in weitere Produkte der Formel I umgewandelt werden können.

Sofern nicht speziell genannt, liegt bei der Nennung einer Verbindung der Formel I stets ein Gemisch beider enantiomeren Formen vor. Beide Antipoden zeigen unterschiedliche mikrobizide Aktivität.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemiöeia, Rhizoctonia, Pellicularia, Puccinia); Fungi imperfecti (z.B. Botrytis, Helminthosporium, Fusarium, Septoria, Cercospora, Piricularia und Alternaria) und gegen Phytomyceten wie Pythium. Verbindungen der Formel I wirken auch gegen phytopathogene Bakterien, wie insbesondere gegen die zur Familie der Pseudomonadaceae gehörenden Xanthomonas Species. Überdies wirken die Verbindungen der Formel I systemisch. Sie können ferner als Beizmittel zur Behandlung von

Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus. Verbindungen der Formel I zeigen auch eine mikrobizide Aktivität gegen Pilzarten im Vorratsschutzbereich, so z.B. gegen verschiedene Schimmelpilze und einige Hefen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einer oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Salat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde-, Düngemitteln oder auch Phospholipiden tierischer oder pflanzlicher Herkunft. Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Applikation solcher Mittel kann direkt auf die Pflanze oder Pflanzenteile erfolgen (Blattapplikation) oder auf den Standort der Pflanze (Bodenapplikation) oder auf die Vermehrungsstelle, z.B. durch Saatgut-Applikation.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden). Solche Mittel sind gleichfalls Gegenstand vorliegender Erfindung.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethyl-ether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl; oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht sorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

«Mc Cutcheon's Detergents and Emulsifiers Annual» MC,
Publishing Corp., Ringwood, New Jersey, 1980
Sisely and Wood, «Encyclopedia of Surface Active Agents»,
Chemical Publishing Co, Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% fester oder flüssiger Zusatzstoffe, darunter 0 bis 25%, insbesondere 0,1 bis 25% eines oder mehrerer Tenside.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs. = absolut wasserfrei; DMSO = Dimethylsulfoxid; DMF = Dimethylformamid; THF = Tetrahydrofuran.

Herstellungsbeispiele

Beispiel H1: Herstellung von

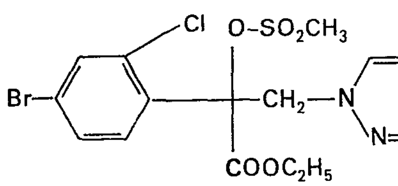

(Verb. Nr. 1.8)

2-(1H-1,2,4-Triazolylmethyl-1'-yl)-2-methylsulfonyloxy-2-chlor-4-bromphenylessigsäureethylester

1,8 g (0,04 Mol) 55%ige Natriumhydrid-Dispersion in Paraffinöl wurden in 50 ml THF vorgelegt und 15 g (0,04 Mol) 2-(1H-1,2,4-Triazolylmethyl-1'-yl)-2-hydroxy-2-chlor-4-bromphenylessigsäureethylester, gelöst in 70 ml THF, bei RT unter Stickstoffatmosphäre zugetropft. Nach 2 h, nachdem die Wasserstoffentwicklung beendet war, wurden 5,5 g (0,048 Mol) Methansulfochlorid bei 0–5°C zugetropft und das Reaktionsgemisch über Nacht bei RT gerührt. Nach dem Einengen im Wasserstrahlvakuum wurde der Rückstand in Diethylether aufgenommen und nacheinander mit Sodalösung und Wasser gewaschen, mit Natriumsulfat getrocknet, filtriert und eingeengt. Das Rohprodukt wurde säulenchromatographisch (Kieselgel/Diethylether) gereinigt. Man erhielt auf diese Weise 7,7 g 2-(1H-1,2,4-Triazolylmethyl-1'-yl)-2-methylsulfonyloxy-2-chlor-4-bromphenylessigsäureethylester.
Smp. 105–113°C.

Beispiel H2: Herstellung von

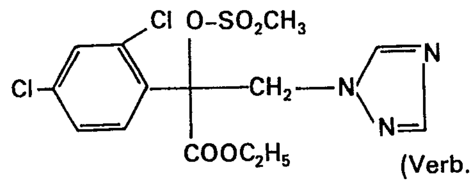

(Verb. Nr. 1.62)

2-(1H-1,2,4-Triazolylmethyl-1'-yl)-2-methylsulfonyloxy-2,4-dichlorphenylessigsäureethylester

6,6 g (0,02 Mol) 2-(1H-1,2,4-Triazolylmethyl-1'-yl)-2-hydroxy-2,4-dichlorphenylessigsäureethylester, 4,1 ml Triethylamin und 0,5 g 4-Dimethylamino-pyridin wurden in 40 ml absolutem Dichlormethan vorgelegt. Zu dieser Mischung wurden unter Eiskühlung und Rühren innerhalb von 15 min 2,5 ml Methansulfochlorid, gelöst in 10 ml Dichlormethan, zugetropft und das Reaktionsgemisch bei RT 6 h gerührt. Anschliessend wurde wieder mit Eiswasser gekühlt, Eiswasser zugetropft, und mit Dichlormethan extrahiert. Die vereinigten Extrakte wurden mit wenig Bicarbonatlösung und anschliessend mit Wasser gewaschen, über Natriumsulfat getrocknet, filtriert und eingedampft. Das resultierende Öl wurde säulenchromatographisch (Kieselgel/Dichlormethan-Isopropanol) gereinigt. Man erhielt 7,5 g (92,6% d.Th.) des 2-(1H-1,2,4-Triazolylmethyl-1'-yl)-2-methylsulfonyloxy-2,4-dichlorphenylessigsäureethylesters. Smp. 119–121°C.

Beispiel H3: Herstellung von

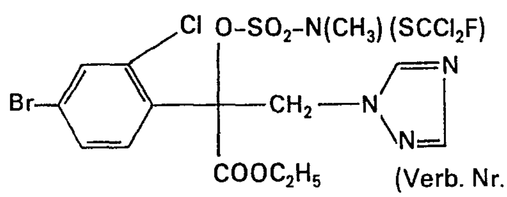

(Verb. Nr. 1.43)

2-(1H-1,2,4-Triazolylmethyl-1'-yl)-2-(N-methyl-N-fluor-dichlormethansulfenyl-sulfamoyloxy)-2-chlor-4-bromphenylessigsäureethylester

6,1 g (0,013 Mol) 2-(1H-1,2,4-Triazolylmethyl-1'-yl)-2-(N-methylsulfamoyloxy)-2-chlor-4-bromphenylessigsäureethylester wurden in 100 ml Essigsäureethylester gelöst und nacheinander mit 4,6 g (0,0273 Mol) Fluordichlormethansulfenylchlorid und 2,9 g (0,0286 Mol) Triethylamin bei 0–5°C tropfenweise versetzt. Das Reaktionsgemisch wurde anschliessend bei RT über Nacht gerührt, dann nacheinander mit Wasser, Sodalösung und wieder Wasser gewaschen, mit

Natriumsulfat getrocknet, filtriert und eingeengt. Auf diese Weise erhielt man 8,1 g des obigen Produktes in Form einer harzigen Masse mit einem Schmelzbereich von 40–55°C.

Beispiel H4: Herstellung von

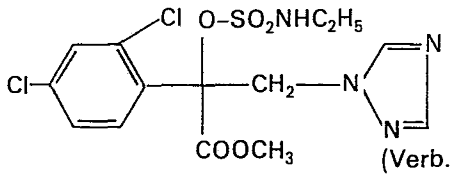

(Verb. Nr. 1.113)

2-(1H-1,2,4-Triazolylmethyl-1′-yl)-2-(N-ethylsulfamoyloxy)-2,4-dichlorphenylessigsäuremethylester

6,2 g (0,02 Mol) 2-(1H-1,2,4-Triazolylmethyl-1′-yl)-2-hydroxy-2,4-dichlorphenylessigsäureethylester wurden zusammen mit 41 ml Triethylamin und 0,5 g 4-Dimethylaminopyridin in 40 ml Dichlormethan suspendiert. Dazu wurden unter Eiskühlung und Rühren bei 5–10° 5,0 ml Ethylsulfamoylchlorid in 10 ml Dichlormethan innerhalb von 20 min zugetropft. Die entstandene Lösung wurde 20 h bei RT gerührt und nach Abkühlen auf 0–5° mit Eiswasser versetzt, mit Bicarbonatlösung neutralisiert und mit Dichlormethan extrahiert. Die Extrakte wurden mit Wasser gewaschen, getrocknet, eingedampft und zunächst aus THF/Hexan, dann aus Essigester umkristallisiert, wobei von wenig unlöslichem Material heiss abfiltriert wurde. Man erhielt 5,5 g (64% d.Th.) des Produktes. Smp. 138–141°C.

Beispiel H5: Herstellung von

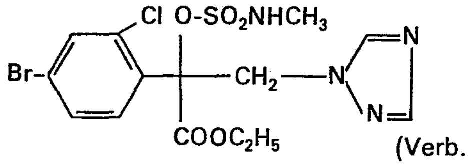

(Verb. Nr. 1.39)

2-(1H-1,2,4-Triazolylmethyl-1′-yl)-2-(N-methylsulfamoyloxy)-2-chlor-4-bromphenylessigsäureethylester

4,4 g (0,1 Mol) 55%ige Natriumhydrid-Dispersion in Paraffinöl wurden unter Stickstoffatmosphäre in 50 ml THF vorgelegt und dazu 27,5 g (0,1 Mol) 2-(1H-1,2,4-Triazolylmethyl-1′-yl)-2-hydroxy-2-chlor-4-bromphenylessigsäureethylester in 130 ml THF bei RT zugetropft. Nach beendeter Wasserstoffentwicklung wurden 16,9 g (0,12 Mol) Methylsulfoamoylchlorid bei 0–5°C zugetropft und das Reaktionsgemisch über Nacht bei RT gerührt. Nach dem Einengen im Wasserstrahlvakuum wurde der Rückstand in Essigsäureethylester gelöst und nacheinander mit verdünnter Sodalösung und Wasser gewaschen, über Natriumsulfat getrocknet, filtriert, eingeengt und durch Zugabe von Diethylether zur Kristallisation gebracht. Durch Filtrieren und Trocknen erhielt man 33 g des obigen Produktes. Smp. 130–145°C.

Beispiel H6: Herstellung von

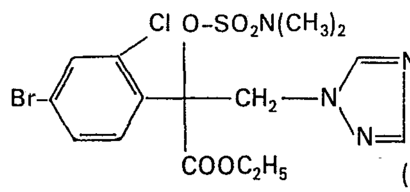

(Verb. Nr. 1.48)

2-(1H-1,2,4-Triazolylmethyl-1′-yl)-2-(N,N-dimethylsulfamoyloxy)-2-chlor-4-bromphenylessigsäureethylester

1,3 g (0,03 Mol) 55%ige Natriumhydrid-Dispersion in Paraffinöl wurden unter Stickstoffatmosphäre in 30 ml THF vorgelegt und bei RT mit 11,2 g (0,03 Mol) 2-(1H-1,2,4-Triazolylmethyl-1′-yl)-2-hydroxy-2-chlor-4-bromphenylessigsäureethylester in 70 ml THF versetzt. Nach beendeter Wasserstoffentwicklung wurden bei 0–5°C 5,2 g (0,036 Mol) Dimethylsulfamoylchlorid zugetropft und das Reaktionsgemisch über Nacht bei RT gerührt. Nach dem Einengen im Wasserstrahlvakuum wurde der Rückstand in Essigsäureethylester aufgenommen, nacheinander mit eiskalter Sodalösung und Wasser gewaschen, über Natriumsulfat getrocknet, filtriert und eingeengt. Zur Abtrennung des Paraffinöls wurde der neue Rückstand in Acetonitril gelöst und das Öl im Scheidetrichter abgetrennt. Nach dem Einengen der Acetonitrillösung im Vakuum erhielt man 11,3 g des obigen Produktes als harzige Masse.

Nach Art der vorstehenden Beispiele und der einleitend angegebenen Herstellungsvarianten lassen sich folgende Verbindungen gemäss vorliegender Erfindung herstellen:

Tabelle 1: Verbindungen der Formel

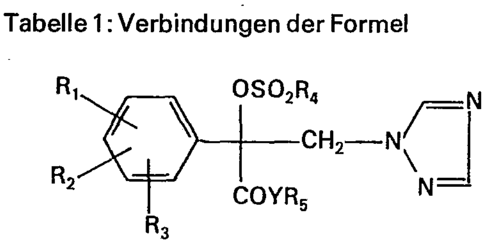

| Verb. Nr. | $R_1$ | $R_2$ | $R_3$ | $R_4$ | $-YR_5$ | X | Phys. Daten |
|---|---|---|---|---|---|---|---|
| 1.1 | H | H | H | $CH_3$ | $OC_2H_5$ | N | |
| 1.2 | H | H | H | $NHCH_3$ | $OC_2H_5$ | N | |

Tabelle 1: (Fortsetzung)

| Verb. Nr. | $R_1$ | $R_2$ | $R_3$ | $R_4$ | $-YR_5$ | X | Phys. Daten |
|---|---|---|---|---|---|---|---|
| 1.3 | H | H | H | 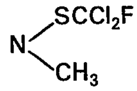 | $OC_2H_5$ | N | |
| 1.4 | 4-Cl | H | H | $CH_3$ | $OC_2H_5$ | N | Smp. 71-77°C |
| 1.5 | 4-Cl | H | H | $NH-CH_3$ | $OC_2H_5$ | N | Smp. 85-99°C |
| 1.6 | 4-Cl | H | H | 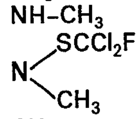 | $OC_2H_5$ | N | $n_D^{40}$ 1,5501 |
| 1.7 | 2-Cl | 4-Br | H | $CH_3$ | $OCH_3$ | N | |
| 1.8 | 2-Cl | 4-Br | H | $CH_3$ | $OC_2H_5$ | N | Smp. 105-113°C |
| 1.9 | 2-Cl | 4-Br | H | $CH_3$ | $OC_3H_7(i)$ | N | $n_D^{40}$ 1,5430 |
| 1.10 | 2-Cl | 4-Br | H | $CH_3$ | $O-C_6H_4Cl(4)$ | N | |
| 1.11 | 2-Cl | 4-Br | H | $CH_3$ | $SC_2H_5$ | N | |
| 1.12 | 2-Cl | 4-Br | H | $CH_3$ | $NHC_2H_5$ | N | |
| 1.13 | 2-Cl | 4-Br | H | $CH_3$ | NH-Benzyl | N | |
| 1.14 | 2-Cl | 4-Br | H | $CH_3$ | 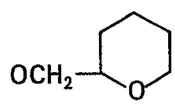 | N | |
| 1.15 | 2-Cl | 4-Br | H | $CH_3$ | O-Benzyl | N | |
| 1.16 | 2-Cl | 4-Br | H | $CH_3$ | $OCH_2CH=CH_2$ | N | |
| 1.17 | 2-Cl | 4-Br | H | $CH_3$ | $OCH_2C\equiv CH$ | N | |
| 1.18 | 2-Cl | 4-Br | H | $CH_3$ | 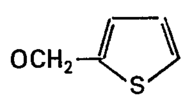 | N | |
| 1.19 | 2-Cl | 4-Br | H | $CH_3$ | $NH_2$ | N | |
| 1.20 | 2-Cl | 4-Br | H | $CH_3$ | $OCH_3$ | CH | |
| 1.21 | 2-Cl | 4-Br | H | $CH_3$ | $OCH_2CH_2OCH_3$ | N | |
| 1.22 | 2-Cl | 4-Br | H | $NHCH_3$ | $OCH_2CH_2OCH_3$ | N | |
| 1.23 | 2-Cl | 4-Br | H | 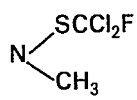 | $OCH_2CH_2OCH_3$ | N | |
| 1.24 | 2-Cl | 4-Br | H | 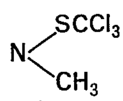 | $OCH_2CH_2OCH_3$ | N | |
| 1.25 | 2-Cl | 4-Br | | $CH_3$ | $OCH_2CH_2Cl$ | N | |
| 1.26 | 2-Cl | 4-Br | H | $NHCH_3$ | $OCH_2CH_2Cl$ | N | |
| 1.27 | 2-Cl | 4-Br | H | 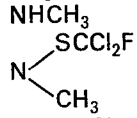 | $OCH_2CH_2Cl$ | N | |
| 1.28 | 2-Cl | 4-Br | H | 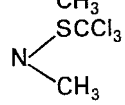 | $OCH_2CH_2Cl$ | N | |
| 1.29 | 2-Cl | 4-Br | H | $CH_3$ | 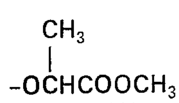 | N | |
| 1.30 | 2-Cl | 4-Br | H | $CH_3$ | $-OCH_2SCH_3$ | N | |
| 1.31 | 2-Cl | 4-Br | H | $CH_3$ | $-OCH_2CO-C_4H_3-t$ | N | |
| 1.32 | 2-Cl | 4-Br | H | $CH_3$ | $-OCH_2OCH_3$ | N | |
| 1.33 | 2-Cl | 4-Br | H | $CH_3$ | $OC_{12}H_{25}(n)$ | N | |
| 1.34 | 2-Cl | 4-Br | H | $C_2H_5$ | $OC_2H_5$ | N | $n_D^{39}$ 1,5449 |
| 1.35 | 2-Cl | 4-Br | H | $C_3H_7(n)$ | $OC_2H_5$ | N | $n_D^{40}$ 1,5392 |

Tabelle 1: (Fortsetzung)

| Verb. Nr. | $R_1$ | $R_2$ | $R_3$ | $R_4$ | $-YR_5$ | X | Phys. Daten |
|---|---|---|---|---|---|---|---|
| 1.36 | 2-Cl | 4-Br | H | $CH_2Cl$ | $OC_2H_5$ | N | Smp. 50-65°C |
| 1.37 | 2-Cl | 4-Br | H | $C_6H_4CH_3(4)$ | $OC_2H_5$ | N | Smp. 50-70°C |
| 1.38 | 2-Cl | 4-Br | H | $-CH_2C_6H_4CH_3(4)$ | $OC_2H_5$ | N | |
| 1.39 | 2-Cl | 4-Br | H | $NHCH_3$ | $OC_2H_5$ | N | Smp. 130-145°C |
| 1.40 | 2-Cl | 4-Br | H | $NHC_2H_5$ | $OC_2H_5$ | N | Smp. 126-130°C |
| 1.41 | 2-Cl | 4-Br | H | $NHC_3H_7(n)$ | $OC_2H_5$ | N | |
| 1.42 | 2-Cl | 4-Br | H | $NHC_4H_9(n)$ | $OC_2H_5$ | N | |
| 1.43 | 2-Cl | 4-Br | H | 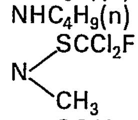 | $OC_2H_5$ | N | Smp. 40-55°C |
| 1.44 | 2-Cl | 4-Br | H | 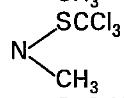 | $OC_2H_5$ | N | Smp. 55-65°C |
| 1.45 | 2-Cl | 4-Br | H | 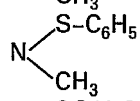 | $OC_2H_5$ | N | |
| 1.46 | 2-Cl | 4-Br | H | 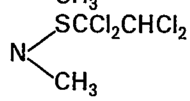 | $OC_2H_5$ | N | |
| 1.47 | 2-Cl | 4-Br | H | 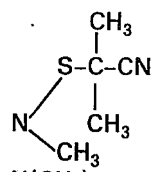 | $OC_2H_5$ | N | |
| 1.48 | 2-Cl | 4-Br | H | $N(CH_3)_2$ | $OC_2H_5$ | N | $n_D^{39}$ 1,5379 |
| 1.49 | 2-Cl | 4-Br | H | $CH_3$ | $OC_2H_5$ | CH | Smp. 51-77°C |
| 1.50 | 2-Cl | 4-Br | H | $NHCH_3$ | $OC_2H_5$ | CH | |
| 1.51 | 2-Cl | 4-Br | H | 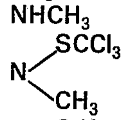 | $OC_2H_5$ | CH | |
| 1.52 | 2-Cl | 4-Br | H | 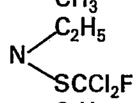 | $OC_2H_5$ | N | $n_D^{39}$ 1,5419 |
| 1.53 | 2-Cl | 4-Br | H | 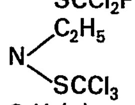 | $OC_2H_5$ | N | $n_D^{41}$ 1,5650 |
| 1.54 | 2-Cl | 4-Br | H | $C_4H_9(n)$ | $OC_2H_5$ | N | |
| 1.55 | 2-Cl | 4-Br | H | $C_{12}H_{25}(n)$ | $OC_2H_5$ | N | |
| 1.56 | 2-Cl | 4-Br | H | $C_2H_5$ | $OC_3H_7(i)$ | N | |
| 1.57 | 2-Cl | 4-Br | H | $CH_3$ | $OC_4H_9(n)$ | N | |
| 1.58 | 2-Cl | 4-Br | H | $CH_3$ | 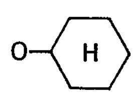 | N | |
| 1.59 | 2-Cl | 4-Br | H | $CH_3$ | 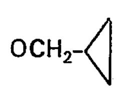 | N | |
| 1.60 | 2-Cl | 4-Cl | H | $CH_3$ | $OCH_3$ | N | Smp. 168-170°C |
| 1.61 | 2-Cl | 4-Cl | H | $CH_3$ | $OCH_3$ | CH | |
| 1.62 | 2-Cl | 4-Cl | H | $CH_3$ | $OC_2H_5$ | N | Smp. 119-121°C |
| 1.63 | 2-Cl | 4-Cl | H | $CH_3$ | $OC_2H_5$ | CH | |
| 1.64 | 2-Cl | 4-Cl | H | $CH_3$ | $OC_3H_7(n)$ | N | |
| 1.65 | 2-Cl | 4-Cl | H | $CH_3$ | $OC_3H_7(i)$ | N | |
| 1.66 | 2-Cl | 4-Cl | H | $CH_3$ | $OC_4H_9(n)$ | N | $n_D^{54}$ 1,5262 |
| 1.67 | 2-Cl | 4-Cl | H | $CH_3$ | $OC_{12}H_{25}(n)$ | N | |

Tabelle 1: (Fortsetzung)

| Verb. Nr. | $R_1$ | $R_2$ | $R_3$ | $R_4$ | $-YR_5$ | X | Phys. Daten |
|---|---|---|---|---|---|---|---|
| 1.68 | 2-Cl | 4-Cl | H | $CH_3$ | $OCH_2$- 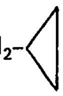 | N | |
| 1.69 | 2-Cl | 4-Cl | H | $CH_3$ | $OCH_2CH_2Cl$ | N | |
| 1.70 | 2-Cl | 4-Cl | H | $CH_3$ | $OCH_2CH_2OCH_3$ | N | |
| 1.71 | 2-Cl | 4-Cl | H | $CH_3$ | $OCH_2OCH_3$ | N | |
| 1.72 | 2-Cl | 4-Cl | H | $CH_3$ | $OCH_2SCH_3$ | N | |
| 1.73 | 2-Cl | 4-Cl | H | $CH_3$ | $OCH_2CH_2SCH_3$ | N | |
| 1.74 | 2-Cl | 4-Cl | H | $CH_3$ | O- 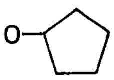 | N | |
| 1.75 | 2-Cl | 4-Cl | H | $CH_3$ | O- 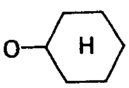 | N | |
| 1.76 | 2-Cl | 4-Cl | H | $CH_3$ | O-Benzyl | N | |
| 1.77 | 2-Cl | 4-Cl | H | $CH_3$ | $O-C_6H_4Cl(4)$ | N | |
| 1.78 | 2-Cl | 4-Cl | H | $CH_3$ | $OCH_2CH=CH_2$ | N | |
| 1.79 | 2-Cl | 4-Cl | H | $CH_3$ | $OCH_2C\equiv CH$ | N | |
| 1.80 | 2-Cl | 4-Cl | H | $CH_3$ | $OCH(CH_3)COOCH_3$ | N | |
| 1.81 | 2-Cl | 4-Cl | H | $CH_3$ | $OCH_2$- 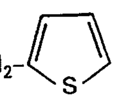 | N | |
| 1.82 | 2-Cl | 4-Cl | H | $CH_3$ | O- 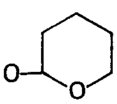 | N | |
| 1.83 | 2-Cl | 4-Cl | H | $CH_3$ | 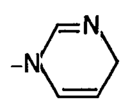 | N | |
| 1.84 | 2-Cl | 4-Cl | H | $CH_3$ | 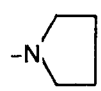 | N | |
| 1.85 | 2-Cl | 4-Cl | H | $CH_3$ | $OCH_2CCl_3$ | N | |
| 1.86 | 2-Cl | 4-Cl | H | $CH_3$ | $-S-C_6H_4C_4H_9-t(4)$ | N | |
| 1.87 | 2-Cl | 4-Cl | H | $CH_3$ | $-OCH(C_3H_7(i))_2$ | N | |
| 1.88 | 2-Cl | 4-Cl | | | $NH_2$ | N | |
| 1.89 | 2-Cl | 4-Cl | H | $CH_3$ | $NHC_2H_5$ | N | |
| 1.90 | 2-Cl | 4-Cl | H | $CH_3$ | $-NH-C_6H_5$ | N | |
| 1.91 | 2-Cl | 4-Cl | H | $C_2H_5$ | $-O-C_6H_4Cl(4)$ | N | |
| 1.92 | 2-Cl | 4-Cl | H | $CH_3$ | -NH-Benzyl | N | |
| 1.93 | 2-Cl | 4-Cl | H | $C_3H_7-n$ | $-S-C_6H_4CH_3(4)$ | N | |
| 1.94 | 2-Cl | 4-Cl | H | $C_4H_9-n$ | $-O-C_4H_9-t$ | N | |
| 1.95 | 2-Cl | 4-Cl | H | $CH_3$ | $SC_2H_5$ | N | |
| 1.96 | 2-Cl | 4-Cl | H | $CH_3$ | $SCH_2$- 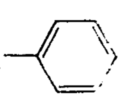 | N | |

Tabelle 1: (Fortsetzung)

| Verb. Nr. | $R_1$ | $R_2$ | $R_3$ | $R_4$ | $-YR_5$ | X | Phys. Daten |
|---|---|---|---|---|---|---|---|
| 1.97 | 2-Cl | 4-Cl | H | $C_2H_5$ | $OCH_3$ | N | |
| 1.98 | 2-Cl | 4-Cl | H | $C_2H_5$ | $OCH_5$ | N | |
| 1.99 | 2-Cl | 4-Cl | H | $C_2H_5$ | $OC_3H_7(i)$ | N | |
| 1.100 | 2-Cl | 4-Cl | H | $C_2H_5$ | $OC_4H_9(n)$ | N | |
| 1.101 | 2-Cl | 4-Cl | H | $CH_2Cl$ | $OCH_3$ | N | |
| 1.102 | 2-Cl | 4-Cl | H | $C_3H_7(n)$ | $OCH_3$ | N | |
| 1.103 | 2-Cl | 4-Cl | H | $C_4H_9(n)$ | $OCH_3$ | N | |
| 1.104 | 2-Cl | 4-Cl | H | 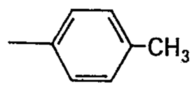 | $OCH_3$ | N | |
| 1.105 | 2-Cl | 4-Cl | H | 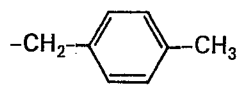 | $OCH_3$ | N | |
| 1.106 | 2-Cl | 4-Cl | H | $-C_3H_7(i)$ | $OCH_3$ | N | |
| 1.107 | 2-Cl | 4-Cl | H | $-NHCH_3$ | $OCH_3$ | N | |
| 1.108 | 2-Cl | 4-Cl | H | 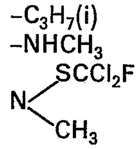 | $OCH_3$ | N | |
| 1.109 | 2-Cl | 4-Cl | H | 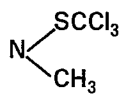 | $OCH_3$ | N | |
| 1.110 | 2-Cl | 4-Cl | H | 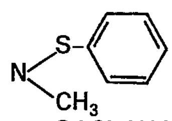 | $OCH_3$ | N | |
| 1.111 | 2-Cl | 4-Cl | H | 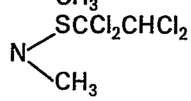 | $OCH_3$ | N | |
| 1.112 | 2-Cl | 4-Cl | H | 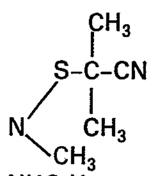 | $OCH_3$ | N | |
| 1.113 | 2-Cl | 4-Cl | H | $NHC_2H_5$ | $OCH_3$ | N | Smp. 138–141°C |
| 1.114 | 2-Cl | 4-Cl | H | 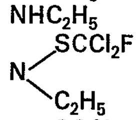 | $OCH_3$ | N | |
| 1.115 | 2-Cl | 4-Cl | H | 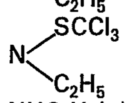 | $OCH_3$ | N | |
| 1.116 | 2-Cl | 4-Cl | H | $NHC_3H_7(n)$ | $OCH_3$ | N | |
| 1.117 | 2-Cl | 4-Cl | H | $NHC_4H_9(n)$ | $OCH_3$ | N | |
| 1.118 | 2-Cl | 4-Cl | H | $NHCH_3$ | $OC_2H_5$ | N | |
| 1.119 | 2-Cl | 4-Cl | H | 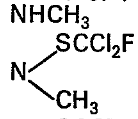 | $OC_2H_5$ | N | |
| 1.120 | 2-Cl | 4-Cl | H | 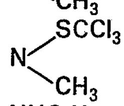 | $OC_2H_5$ | N | |
| 1.121 | 2-Cl | 4-Cl | H | $NHC_2H_5$ | $OC_2H_5$ | N | |
| 1.122 | 2-Cl | 4-Cl | H | $NHC_2H_5$ | $OC_3H_7(n)$ | N | |
| 1.123 | 2-Cl | 4-Cl | H | $NHC_2H_5$ | $OC_4H_9(n)$ | N | |

Tabelle 1: (Fortsetzung)

| Verb. Nr. | $R_1$ | $R_2$ | $R_3$ | $R_4$ | $-YR_5$ | X | Phys. Daten |
|---|---|---|---|---|---|---|---|
| 1.124 | 2-Cl | 4-Cl | H | $NHCH_3$ | $SC_2H_5$ | N | |
| 1.125 | 2-Cl | 4-Cl | H | $NHCH_3$ | $N(CH_3)_2$ | N | |
| 1.126 | 2-Cl | 4-Cl | H | $CH_3$ | $OCH_2COC_4H_9(t)$ | N | |
| 1.127 | 2-Cl | 4-Cl | H | $CH_3$ | $OCH_2CO-C_6H_3Cl_2(2,4)$ | N | |
| 1.128 | 2-Cl | 4-Cl | H | $NHCH_3$ | $OC_3H_7(i)$ | N | |
| 1.129 | 2-Cl | 4-Cl | H | $NHCH_3$ | $OCH_3$ | CH | |
| 1.130 | 2-Cl | 4-Cl | H | $NHCH_3$ | $OC_4H_9(n)$ | N | |
| 1.131 | 2-Cl | 4-Cl | H | $C_2H_5$ | 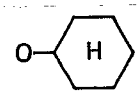 | N | |
| 1.132 | 3-Cl | 4-Cl | H | $CH_3$ | $OC_2H_5$ | N | |
| 1.133 | 3-Cl | 4-Cl | H | $C_2H_5$ | $OC_2H_5$ | N | |
| 1.134 | 3-Cl | 4-Cl | H | $NHCH_3$ | $OC_2H_5$ | N | |
| 1.135 | 3-Cl | 4-Cl | H | 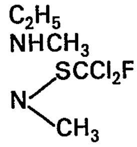 | $OC_2H_5$ | N | |
| 1.136 | 2-Cl | 4-Cl | 6-Cl | $CH_3$ | $OC_2H_5$ | N | |
| 1.137 | 2-Cl | 4-Cl | 6-Cl | $C_2H_5$ | $OC_2H_5$ | N | |
| 1.138 | 2-Cl | 4-Cl | 6-Cl | $NHCH_3$ | $OC_2H_5$ | N | |
| 1.139 | 2-Cl | 4-Cl | 6-Cl | 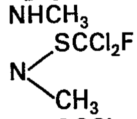 | $OC_2H_5$ | N | |
| 1.140 | 2-Cl | 4-Cl | 6-Cl | 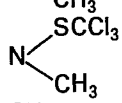 | $OC_2H_5$ | N | |
| 1.141 | 2-Br | 4-Br | H | $CH_3$ | $OC_2H_5$ | N | Smp. 40–52°C |
| 1.142 | 2-Br | 4-Br | G | $C_2H_5$ | $OC_2H_5$ | N | |
| 1.143 | 2-Br | 4-Br | H | $NHCH_3$ | $OC_2H_5$ | N | Smp. 140–144°C |
| 1.144 | 2-Br | 4-Br | H | 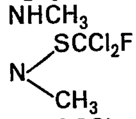 | | | |
| 1.145 | 2-Br | 4-Br | H | 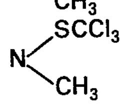 | $OC_2H_5$ | N | |
| 1.146 | 4-Br | H | H | $CH_3$ | $OCH_3$ | N | |
| 1.147 | 4-Br | H | H | $C_2H_5$ | $OCH_3$ | N | |
| 1.148 | 4-Br | H | H | $NHCH_3$ | $OCH_3$ | N | |
| 1.149 | 2-Cl | 4-F | H | $CH_3$ | $OCH_3$ | N | |
| 1.150 | 2-Cl | 4-F | H | $CH_3$ | $OC_2H_5$ | N | |
| 1.151 | 2-Cl | 4-F | H | $CH_3$ | $OC_3H_7(i)$ | N | |
| 1.152 | 2-Cl | 4-F | H | $CH_3$ | $OC_4H_9(n)$ | N | |
| 1.153 | 2-Cl | 4-F | H | $C_2H_5$ | $OCH_3$ | N | |
| 1.154 | 2-Cl | 4-F | H | $C_2H_5$ | $OC_2H_5$ | N | |
| 1.155 | 2-Cl | 4-F | H | $NHCH_3$ | $OC_2H_5$ | N | |
| 1.156 | 2-Cl | 4-F | H | $NHCH_3$ | $OCH_3$ | N | |
| 1.157 | 2-Cl | 4-F | H | 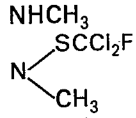 | $OCH_3$ | N | |
| 1.158 | 2-Cl | 4-F | H | $NHC_2H_5$ | $OCH_3$ | N | |
| 1.159 | 2-Cl | 4-F | H | $NHC_3H_7(n)$ | $OCH_3$ | N | |
| 1.160 | 2-Cl | 4-F | H | $NHC_4H_9(n)$ | $OCH_3$ | N | |
| 1.161 | 2-Cl | 4-F | H | $C_4H_9(n)$ | $OCH_3$ | N | |
| 1.162 | 2-Cl | 4-F | G | $CH_3$ | 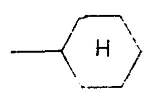 | N | |

Tabelle 1: (Fortsetzung)

| Verb. Nr. | $R_1$ | $R_2$ | $R_3$ | $R_4$ | $-YR_5$ | X | Phys. Daten |
|---|---|---|---|---|---|---|---|
| 1.163 | 2-Cl | 4-F | H | $CH_3$ | $OCH_2CH_2OCH_3$ | N | |
| 1.164 | 2-Cl | 4-F | H | $CH_3$ | $OCH_2CH_2Cl$ | N | |
| 1.165 | 2-Cl | 4-F | G | $CH_3$ | $SC_2H_5$ | N | |
| 1.166 | 2-Cl | 4-F | H | $CH_3$ | $NH_2$ | N | |
| 1.167 | 2-Cl | 4-F | H | $CH_3$ | 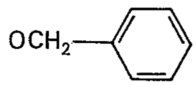 | N | |
| 1.168 | 2-$CH_3$ | 5-$CH_3$ | H | $CH_3$ | $OCH_3$ | N | |
| 1.169 | 3-$CF_3$ | H | H | $CH_3$ | $OCH_3$ | N | |
| 1.170 | 3-$NO_2$ | H | H | $CH_3$ | $OCH_3$ | N | |
| 1.171 | 4-CN | H | H | $CH_3$ | $OCH_3$ | N | |
| 1.172 | 4-$CH_3O$ | H | H | $CH_3$ | $OCH_3$ | N | |
| 1.173 | 2-Cl | 4-F | H | $CH_3$ | $OCH_3$ | CH | |
| 1.174 | 2-Cl | 4-Cl | H | $N(CH_3)_2$ | $OCH_3$ | N | |
| 1.175 | 2-Cl | 4-Cl | H | $-CH_3$ | $-O-C(CH_3)_3$ | N | |
| 1.176 | 2-Cl | 4-Cl | H | $-NHC_2H_5$ | $-OC(CH_3)_3$ | N | |
| 1.177 | 2-Cl | 4-Cl | H | 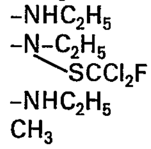 | $-O-C\,(CH_3)_3$ | N | |
| 1.178 | 2-Cl | 4-Cl | H | $-NHC_2H_5$ | $-O-CH_2CH=CH_2$ | N | |
| 1.179 | 2-Br | 4-Br | H | $CH_3$ | $-OC_3H_7$-I | N | Smp. 50-55°C |

Tabelle 2: Verbindungen der Formel

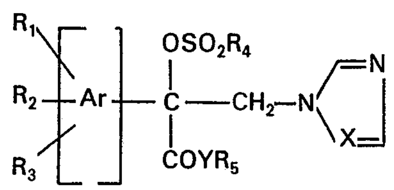

| Verb. Nr. | 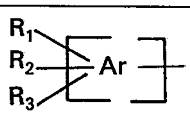 | $R_4$ | $YR_5$ | X | Phys. Daten |
|---|---|---|---|---|---|
| 2.1 | 4-Bipgneyl | $CH_3$ | $OCH_3$ | N | |
| 2.2 | 4-Biphenyl | $CH_3$ | $OCH_3$ | CH | |
| 2.3 | 4-Biphenyl | $CH_3$ | $OC_2H_5$ | N | |
| 2.4 | 4-Biphenyl | $C_2H_5$ | $OCH_3$ | N | |
| 2.5 | 4-Biphenyl | $NHCH_3$ | $OCH_3$ | N | |
| 2.6 | 4-Biphenyl | $NHC_2H_5$ | $OCH_3$ | N | |
| 2.7 | 4-Biphenyl | 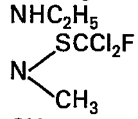 | $OCH_3$ | N | |
| 2.8 | 4-Biphenyl | $CH_3$ | $SC_2H_5$ | N | |
| 2.9 | 4-Biphenyl | $CH_3$ | $O-C_6H_4Cl(4)$ | N | |
| 2.10 | 4-Biphenyl | $CH_3$ | $NHC_2H_5$ | N | |
| 2.11 | 4-Biphenyl | $CH_3$ | $OCH_2CH=CH_2$ | N | |
| 2.12 | 4-Biphenyl | $CH_3$ | $OCH_2=CH$ | N | |
| 2.13 | 4-Biphenyl | $CH_3$ | $O-C(CH_3)_3$ | N | |
| 2.14 | 4-Biphenyl | $CH_3$ | 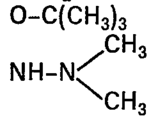 | N | |
| 2.15 | 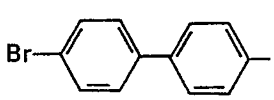 | $CH_3$ | $OCH_3$ | N | |

Tabelle 2: (Fortsetzung)

| Verb. Nr. | | $R_4$ | $YR_5$ | X | Phys. Daten |
|---|---|---|---|---|---|
| 2.16 | 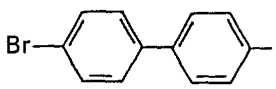 | $CH_3$ | 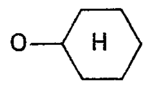 | N | |
| 2.17 | α-Naphthyl | $CH_3$ | $OCH_3$ | N | |
| 2.18 | α-Naphthyl | $NH{-}CH_3$ | $OCH_3$ | N | |
| 2.19 | α-Naphthyl-2-$CH_3$ | $CH_3$ | $OCH_3$ | N | |
| 2.20 | β-Naphthyl | $CH_3$ | $OCH_3$ | N | |
| 2.21 | β-Naphtyhl | $CH_3$ | $OC_2H_5$ | N | |
| 2.22 | β-Naphthyl | $C_2H_5$ | $OCH_3$ | N | |
| 2.23 | β-Naphthyl | $NHCH_3$ | $OCH_3$ | N | |
| 2.24 | β-Naphthyl | $NHC_2H_5$ | $OCH_3$ | N | |
| 2.25 | β-Naphthyl | 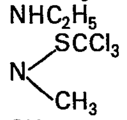 | $OCH_3$ | N | |
| 2.26 | 4-Phenoxyphenyl | $CH_3$ | $OCH_3$ | N | |
| 2.27 | 4-Phenoxyphenyl | $C_2H_5$ | $OC_2H_5$ | N | |
| 2.28 | 4-Phenoxyphenyl | $CH_3$ | $OCH_3$ | CH | |

Formulierungsbeispiele für Wirkstoffe der Formel I
(% = Gewichtsprozent)

A. Emulsions-Konzentrate

| | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | – | – |
| Tributylphenoyl-polyethylenglykolether (30 Mol Ethylenoxid) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| B. Lösungen | a) | b) | c) | d) |
|---|---|---|---|---|
| Wirkstoff aus den Tabellen | 80% | 10% | 5% | 95% |
| Ethylenglykol-mono-methyl-ether | 20% | – | – | – |
| Polyethylenglykol M G 400 | – | 70% | – | – |
| N-Methyl-2-pyrrolidon | – | 20% | – | – |
| Epoxydiertes Kokosnussöl | – | – | 1% | 5% |
| Benzin (Siedegrenzen 160–190°C) | – | – | 94% | – |

(MG = Molekulargewicht)

Die Lösungen sind zur Anwendung in Form kleinster Tropfen geeignet.

| C. Granulate | a) | b) |
|---|---|---|
| Wirkstoff aus den Tabellen | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| D. Stäubemittel | a) | b) |
|---|---|---|
| Wirkstoff aus den Tabellen | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

| E. Spritzpulver | a) | b) | c) |
|---|---|---|---|
| Wirkstoff aus den Tabellen | 25% | 50% | 75% |
| Na-Ligninsulfonat | 5% | 5% | – |
| Na-Laurylsulfat | 3% | – | 5% |
| Na-Diisobutylnaphthalin-sulfonat | – | 6% | 10% |
| Octylphenolpolyethylen-glykolether (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut

vermischt und in einer geeigneten Mühle gut vermahlen. Man erhält Spritzpulver, die sich mit Wasser zu Suspensionen jeder gewünschten Konzentration verdünnen lassen.

Biologische Beispiele:

Beispiel B1:
Wirkung gegen Puccinia graminis auf Weizen
a) Residual-protektive Wirkung
Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95–100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung
Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95–100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Unbehandelte, aber infizierte Kontrollpflanzen zeigten einen Befall von 100%. Pflanzen, die mit Mitteln enthaltend Verbindungen der Formel I behandelt waren, zeigten nur geringen (<20%) oder keinen Befall. Die Verbindungen Nr. 1.4, 1.5, 1.6, 1.8, 1.9, 1.34–1.37, 1.39, 1.40, 1.43, 1.44, 1.48, 1.49, 1.52. 1.60, 1.62, 1.66, 1.113, 1.141, 1.143 und 1.179 unterdrückten den Pilzbefall auch noch bei einer Konzentration von 0,002% vollständig.

Beispiel B2:
Wirkung gegen Cercospora arachidicola auf Erdnusspflanzen
Residual-protektive Wirkung
10–15 cm hohe Erdnusspflanzen wurden mit einer aus Spritzpulver der Wirksubstanz hergestellten Spritzbrühe (0,006 Aktivsubstanz besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 und 2 behandelt wurden, einen stark reduzierten Cercospora-Befall. So verhinderten die Verbindungen Nr. 1.8, 1.9, 1.34, 1.35, 1.36, 1.37, 1.39, 1.43, 1.44, 1.60, 1.62, 1.66, 1.113, 1.141 und 1.143 und andere in obigen Versuchen das Auftreten von Flecken fast vollständig (0–10%).

Beispiel B3:
Wirkung gegen Erysiphe graminis auf Gerste
a) Residual-protektive Wirkung
Ca. 8 cm hohe Gerstenpflanzen wurden mit einer aus Spritzpulver des Wirkstoffes hergestellten Spritzbrühe (0,02% Aktivsubstanz) besprüht. Nach 3–4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung
Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I bzw. Verbindungen aus den Tabellen 1 und 2 reduzierten den Pilzbefall unter 20%, während unbehandelte aber infizierte Kontrollpflanzen zu 100% befallen waren. Eine vollständige Hemmung des Pilzbefalls (0–5%) wurde unter anderen mit den Verbindungen Nr. 1.4, 1.5, 1.8, 1.9, 1.34–1.37, 1.39, 1.40, 1.43, 1.44, 1.49, 1.53, 1.60, 1.62, 1.66, 1.113, 1.141, 1.143 und 1.179 erzielt.

Beispiel B4:
Residual-protektive Wirkung gegen Venturia inaequalis auf Apfeltrieben
Apfelstecklinge mit 10–20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24°C aufgestellt. Der Schorfbefall wurde 15 Tage nach der Infektion beurteilt. Verbindungen 1.8, 1.9, 1.36, 1.39, 1.43, 1.44, 1.60, 1.62, 1.66, 1.113 und 1.141 und andere hemmten den Krankheitsbefall auf weniger als 10% und zeigten im Falle der Verbindungen Nr. 1.36 und 1.62 keinen Krankheitsbefall.

Triebe an Apfelbäumen werden im Freiland in gleichem Masse geschützt, ohne in ihrer Weiterentwicklung gehemmt zu werden.

Beispiel B5:

Wirkung gegen Botrytis cinerae auf Bohnen

Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95–100% relativer Luftfeuchtigkeit und 21°C erfolgte die Beurteilung des Pilzbefalls. Die Verbindungen aus den Tabellen 1 und 2 hemmten in vielen Fällen die Pilzinfektion auf weniger als 25%, z.B. die Verbindungen Nr. 1.8, 1.34, 1.35, 1.62, 1.66 und 1.41.

Beispiel B6:

Wirkung gegen Piricularia oryzae auf Reis

Residual-protektive Wirkung

Reispflanzen wurden nach zweiwöchiger Anzucht mit einer aus Spritzpulver des Wirkstoffs hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95–100% relativer Luftfeuchtigkeit und 24°C wurde der Pilzbefall beurteilt.

Gegenüber 100% Befall bei ungeschützten Pflanzen hemmten Verbindungen aus den Tabellen 1 bis 3 den Pilzbefall deutlich, so unterdrückten unter anderen die Verbindungen Nr. 1.8, 1.9 und 1.39 den Piriculariabefall auf weniger als 10%.

**Patentansprüche für die Vertragsstaaten BE, CH, DE, FR, GB, IT, LI, NL, SE**

1. Mandelsäurederivate der Formel I

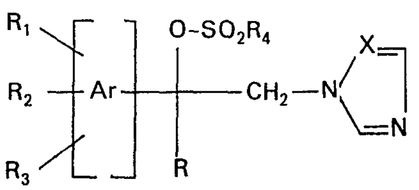

(I),

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$–$C_3$-Alkyl, $C_1$–$C_3$-Alkoxy oder $C_1$–$C_3$-Haloalkyl stehen;

R eine der Gruppen $-COOR_5$, $-COSR_6$,

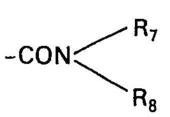

oder -CN darstellt;

$R_5$ ein unsubstituiertes oder durch Halogen substituiertes $C_2$–$C_{10}$-Alkenyl; ein unsubstituiertes oder durch Halogen substituiertes $C_2$–$C_{10}$-Alkinyl; oder eine $C_3$–$C_8$-Cycloalkylgruppe oder eine unsubstituierte oder durch Halogen, $C_1$–$C_4$-Alkyl, $C_1$–$C_4$-Alkoxy, -CN oder $-CF_3$ substituierte Phenylgruppe bedeutet; oder aber eine $C_1$–$C_{12}$-Alkylkette darstellt, die ab $C_2$-Alkyl durch Sauerstoff oder Schwefel unterbrochen und die unsubstituiert oder durch eine der folgenden Atome oder Gruppen substituiert sein kann: Halogen, Phenyl, -COOAlkyl($C_1$–$C_4$), -COAlkyl($C_1$–$C_4$), -COPhenyl, einen ungesättigten oder gesättigten 5- oder 6-gliedrigen Ring mit Sauerstoff oder Schwefel als Heteroatom, wobei jeder Phenylrest unsubstituiert oder ein- oder mehrfach durch gleiche oder verschiedene Halogenatome substituiert ist;

$R_6$ $C_1$–$C_{10}$-Alkyl oder eine unsubstituierte oder durch Halogen, $C_1$–$C_4$-Alkyl, $C_1$–$C_4$-Alkoxy, -CN oder $-CF_3$ substituierte Phenyl- oder Benzylgruppe bedeutet;

$R_7$ und $R_8$ unabhängig voneinander Wasserstoff, $C_1$–$C_6$-Alkyl, $C_3$–$C_7$-Cycloalkyl oder eine Phenyl- oder Benzylgruppe darstellen, bei denen jeweils der aromatische Ring unsubstituiert oder durch Halogen, $C_1$–$C_4$-Alkyl, $C_1$–$C_4$-Alkoxy, -CN oder $-CF_3$ substituiert ist, oder $R_7$ und $R_8$ zusammen einen 5- oder 6gliedrigen gesättigten oder ungesättigten heterocyclischen Ring bilden, der noch 1 oder 2 weitere N-Atome enthalten kann;

$R_4$ gegebenenfalls durch Halogen oder $C_1$–$C_4$-Alkoxy substituiertes $C_1$–$C_{12}$-Alkyl, gegebenenfalls durch Halogen, $C_1$–$C_4$-Alkyl, $C_1$–$C_4$-Alkoxy, $C_1$–$C_4$-Haloalkyl oder Cyano substituiertes Phenyl oder ebenso gegebenenfalls substituiertes Benzyl bedeutet oder für die Gruppe $-N(R_9)(R_{10})$ steht; wobei

$R_9$ $C_1$–$C_6$-Alkyl bedeutet; und

$R_{10}$ für Wasserstoff, $C_1$–$C_4$-Alkyl, $C_1$–$C_4$-Haloalkylsulfenyl, $-SC_6H_5$ oder $-SC(CH_3)_2CN$ steht;

wobei Säureadditionssalze sowie Metallkomplexe der Formel I mit eingeschlossen sind.

2. Mandelsäurederivate nach Anspruch 1 der Formel I*

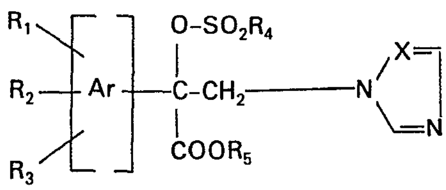

(I*),

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$–$C_3$-Alkyl, $C_1$–$C_3$-Alkoxy oder $C_1$–$C_3$-Haloalkyl stehen;

$R_5$ $C_1$–$C_4$-Alkyl, Phenyl, ein durch Nitro, Halogen und/oder Methyl ein- oder mehrfach substituiertes Phenyl oder Benzyl bedeutet;

$R_4$ für $C_1$–$C_6$-Alkyl, gegebenenfalls durch Fluor, Chlor, Brom, $CF_3$, Methyl, Methoxy oder Cyano substituiertes Phenyl oder ebenso gegebenenfalls substituiertes Benzyl steht oder die Gruppe $-N(R_9)(R_{10})$ repräsentiert; wobei

$R_9$ $C_1$–$C_3$-Alkyl bedeutet; und

$R_{10}$ für Wasserstoff, $C_1$–$C_3$-Alkyl, $-SCCl_3$, $-SCCl_2F$, $-SCCl_2CHCl_2$, $-SC_6H_5$ oder $-SC(CH_3)_2CN$ steht;

unter Einschluss der pflanzenverträglichen Säureadditionsalze, quaternären Azolium- und Ammoniumsalze sowie Metallkomplexe.

3. Mandelsäurederivate der Formel I nach Anspruch 2, worin X für das Brückenglied -CH= oder -N= steht; Ar eine Phenylgruppe bedeutet; $R_1$ in ortho-Position für Wasserstoff, Nitro, Halogen, $C_1$–$C_3$-Alkyl, $C_1$–$C_3$-Alkoxy oder $C_1$–$C_3$-Haloalkyl steht; $R_2$ in para-Position für Wasserstoff, Nitro, Halogen, $C_1$–$C_3$-Alkyl, $C_1$–$C_3$-Alkoxy oder $C_1$–$C_3$-Haloalkyl steht; $R_3$ Wasserstoff, Methyl oder Halogen bedeutet; R eine der Gruppen $-COOR_5$, $-COSR_6$,

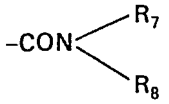

oder -CN darstellt; $R_5$ $C_1$–$C_4$-Alkyl, Phenyl, ein durch Nitro, Halogen und/oder Methyl ein- oder mehrfach substituiertes Phenyl oder ebenso substituiertes Benzyl bedeutet; $R_6$ $C_1$–$C_{10}$-Alkyl oder eine unsubstituierte oder durch Halogen, $C_1$–$C_4$-Alkyl, $C_1$–$C_4$-Alkoxy, -CN oder $-CF_3$ substituierte Phenyl- oder Benzylgruppe bedeutet, $R_7$ und $R_8$ unabhängig voneinander für Wasserstoff, $C_1$–$C_3$-Alkyl, $C_3$–$C_7$-Cycloalkyl, Phenyl oder Benzyl stehen; und $R_4$ für $C_1$–$C_4$-Alkyl, gegebenenfalls durch Fluor, Chlor, Brom, $CF_3$, Methyl oder Methoxy substituiertes Phenyl oder gegebenenfalls ebenso substituiertes Benzyl steht oder die Gruppe $-N(R_9)(R_{10})$ repräsentiert; wobei $R_9$ für $C_1$–$C_3$-Alkyl steht; und $R_{10}$ Wasserstoff, $C_1$–$C_3$-Alkyl, $-SCCl_3$, $-SCCl_2F$, $-SCCl_2CHCl_2$, $-SC_6H_5$ oder $-SC(CH_3)_2CN$ bedeutet; wobei Säureadditionssalze sowie Metallkomplexe der Formel I mit eingeschlossen sind.

4. Mandelsäurederivate der Formel I nach Anspruch 2, worin X für das Brückenglied -N= steht;

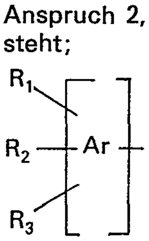

eine in ortho- und/oder para-Position durch Nitro, Fluor, Chlor, Brom, Methyl, Methoxy und/oder $CF_3$ substituierte Phenylgruppe bedeutet; R für die Gruppe $-COOR_5$ steht; $R_5$ $C_1$–$C_4$-Alkyl, Phenyl, ein durch Nitro, Chlor, Brom, Fluor und/oder Methyl substituiertes Phenyl oder ebenso substituiertes Benzyl bedeutet; und $R_4$ für Methyl, Ethyl, Phenyl, p-Tolyl, p-Methylbenzyl, $-NH(C_1$–$C_4$-Alkyl) oder $-N(R_9)(R_{10})$ steht; wobei $R_9$ $C_1$–$C_2$-Alkyl und $R_{10}$ Methyl, $-SCCl_3$, $-SCCl_2F$, $-SCCl_2CHCl_2$, $-SC_6H_5$ oder $-SC(CH_3)_2CN$ bedeuten; wobei Säureadditionssalze sowie Metallkomplexe der Formel I mit eingeschlossen sind.

5. Ein Mandelsäurederivat der Formel I, ausgewählt aus der Reihe:

2-(1H-1,2,4-Triazolylmethyl-1′-yl)-2-methylsulfonyloxy-2-chlor-4-bromphenylessigsäureethylester;

2-(1H-1,2,4-Triazolylmethyl-1′-yl)-2-methylsulfonyloxy-2,4-dichlorphenylessigsäureethylester;

2-(1H-1,2,4-Triazolylmethyl-1′-yl)-2-(N-methyl-N-fluordichlormethansulfenyl-sulfamoyloxy)-2-chlor-4-bromphenylessigsäureethylester;

2-(1H-1,2,4-Triazolylmethyl-1′-yl)-2-(N-ethylsulfamoyloxy)-2,4-dichlorphenylessigsäuremethylester;

2-(1H-1,2,4-Triazolylmethyl-1′-yl)-2-(N-methylsulfamoyloxy)-2-chlor-4-bromphenylessigsäureethylester;

2-(1H-1,2,4-Triazolylmethyl-1′-yl)-2-(N,N-dimethylsulfamoyloxy)-2-chlor-4-bromphenylessigsäureethylester;

2-(1H-1,2,4-Triazolylmethyl-1′-yl)-2-methylsulfonyloxy-4-chlorphenylessigsäureethylester;

2-(1H-1,2,4-Triazolylmethyl-1′-yl)-2-(N-methylsulfonyloxy)-4-chlorphenylessigsäureethylester;

2-(1H-1,2,4-Triazolylmethyl-1′-yl)-2-(N-methyl-N-fluordichlormethansulfenyl-sulfamoyloxy)-4-chlorphenylessigsäureethylester;

2-(1H-1,2,4-Triazolylmethyl-1′-yl)-2-methylsulfonyloxy-2-chlor-4-bromphenylessigsäureisopropylester;

2-(1H-1,2,4-Triazolylmethyl-1′-yl)-2-ethylsulfonyloxy-2-chlor-4-bromphenylessigsäureethylester;

2-(1H-1,2,4-Triazolylmethyl-1′-yl)-2-chlormethylsulfonyloxy-2-chlor-4-bromphenylessigsäureethylester;

2-(1H-1,2,4-Triazolylmethyl-1′-yl)-2-paratosyloxy-2-chlor-4-bromphenylessigsäureethylester;

2-(1H-1,2,4-Triazolylmethyl-1′-yl)-2-(N-trichlormethansulfenylsulfamoyloxy)-2-chlor-4-bromphenylessigsäureethylester;

2-(1H-1,2,4-Triazolylmethyl-1′-yl)-2-methylsulfonyloxy-2,4-dichlorphenylessigsäuremethylester; und

2-(1H-1,2,4-Triazolylmethyl-1′-yl)-2-methylsulfonyloxy-2,4-dichlorphenylessigsäure-n-butylester.

6. Verfahren zur Herstellung der in Anspruch 1 definierten Mandelsäurederivate der Formel I, dadurch gekennzeichnet, dass man einen Alkohol der Formel II

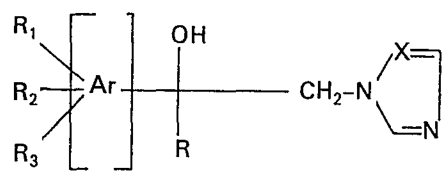
(II)

in An- oder Abwesenheit eines reaktionsinerten organischen Lösungs- oder Verdünnungsmittels, bei Temperaturen von −20°C bis 150°C entweder direkt in Gegenwart einer organischen oder anorganischen Base mit einem Sulfonylhalogenid der Formel XVI

$HalSO_2R_4$ (XVI)

an der OH-Gruppe sulfonyliert oder den Alkohol II vor der Sulfonylierung mit XVI zuerst in ein Alkalialkoholat der Formel III

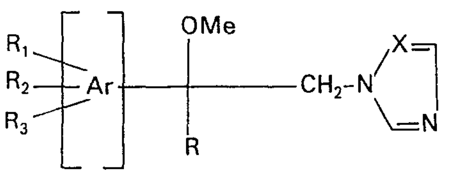

(III)

überführt, wobei die Substituenten in den Formeln II, III und XVI wie unter Formel I definiert sind, Hal für ein Halogenatom steht, und Me ein Alkalimetallatom bedeutet, und, falls erwünscht, die verfahrensgemäss erhältliche Verbindung der Formel I in eine andere Verbindung der Formel I umwandelt und/oder eine verfahrensgemäss erhältliche freie Verbindung in ein Säureadditionssalz umwandelt, ein verfahrensgemäss erhältliches Säureadditionssalz in die freie Verbindung oder ein anderes Säureadditionssalz umwandelt, oder eine verfahrensgemäss erhältliche Verbindung bzw. ein verfahrensgemäss erhältliches Salz in einen Metallkomplex überführt.

7. Verfahren nach Anspruch 6, dadurch gekennzeichnet, dass man die Umsetzung von II mit XVI bei Temperaturen von 0° bis +40°C und von III mit XVI bei Temperaturen von 0° bis +25°C durchführt, wobei der Substituent Hal in Formel XVI für Chlor oder Brom steht und Me in Formel III Natrium oder Kalium bedeutet.

8. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss einem der Ansprüche 1 bis 5 enthält, zusammen mit Trägerstoffen.

9. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 5 auf die Pflanze oder deren Standort appliziert.

10. Verfahren zur Herstellung eines Schädlingsbekämpfungsmittels gemäss Anspruch 10, dadurch gekennzeichnet, dass mindestens eine Verbindung der Formel I nach Anspruch 1 mit geeigneten festen und/oder flüssigen Trägerstoffen und/oder Tensiden innig vermischt wird.

## Patentansprüche für den Vertragsstaat AT

1. Schädlingsbekämpfungsmittel zur Bekämpfung oder Verhütung eines Befalls von Pflanzen durch phytopathogene Mikroorganismen, dadurch gekennzeichnet, dass es neben üblichen Trägermaterialien und applikationsfördernden Zusätzen als aktive Komponente mindestens ein Mandelsäurederivat der Formel

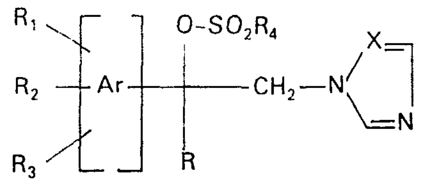

(I),

enthält, worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen;

R eine der Gruppen $-COOR_5$, $-COSR_6$,

$$-CON\begin{matrix} R_7 \\ R_8 \end{matrix}$$

oder -CN darstellt;

$R_5$ ein unsubstituiertes oder durch Halogen substituiertes $C_2$-$C_{10}$-Alkenyl; ein unsubstituiertes oder durch Halogen substituiertes $C_2$-$C_{10}$-Alkinyl; oder eine $C_3$-$C_8$-Cycloalkylgruppe oder eine unsubstituierte oder durch Halogen, $C_1$-$C_4$-Alkyl, $C_1$-$C_4$-Alkoxy, -CN oder $-CF_3$ substituierte Phenylgruppe bedeutet; oder aber eine $C_1$-$C_{12}$-Alkylkette darstellt, die ab $C_2$-Alkyl durch Sauerstoff oder Schwefel unterbrochen und die unsubstituiert oder durch eine der folgenden Atome oder Gruppen substituiert sein kann: Halogen, Phenyl, -COOAlkyl($C_1$-$C_4$), -COAlkyl($C_1$-$C_4$), -COPhenyl, einen ungesättigten oder gesättigten 5- oder 6gliedrigen Ring mit Sauerstoff oder Schwefel als Heteroatom, wobei jeder Phenylrest unsubstituiert oder ein- oder mehrfach durch gleiche oder verschiedene Halogenatome substituiert ist;

$R_6$ $C_1$-$C_{10}$-Alkyl oder eine unsubstituierte oder durch Halogen, $C_1$-$C_4$-Alkyl, $C_1$-$C_4$-Alkoxy, -CN oder $-CF_3$ substituierte Phenyl- oder Benzylgruppe bedeutet;

$R_7$ und $R_8$ unabhängig voneinander Wasserstoff, $C_1$-$C_6$-Alkyl, $C_3$-$C_7$-Cycloalkyl oder eine Phenyl- oder Benzylgruppe darstellen, bei denen jeweils der aromatische Ring unsubstituiert oder durch Halogen, $C_1$-$C_4$-Alkyl, $C_1$-$C_4$-Alkoxy, -CN oder $-CF_3$ substituiert ist, oder $R_7$ und $R_8$ zusammen einen 5- oder 6gliedrigen gesättigten oder ungesättigten heterocyclischen Ring bilden, der noch 1 oder 2 weitere N-Atome enthalten kann;

$R_4$ gegebenenfalls durch Halogen oder $C_1$-$C_4$-Alkoxy substituiertes $C_1$-$C_{12}$-Alkyl, gegebenenfalls durch Halogen, $C_1$-$C_4$-Alkyl, $C_1$-$C_4$-Alkoxy, $C_1$-$C_4$-Haloalkyl oder Cyano substituiertes Phenyl oder ebenso gegebenenfalls substituiertes Benzyl bedeutet oder für die Gruppe $-N(R_9)(R_{10})$ steht; wobei

$R_9$ $C_1$-$C_6$-Alkyl bedeutet; und

$R_{10}$ für Wasserstoff, $C_1$-$C_4$-Alkyl, $C_1$-$C_4$-Haloalkylsulfenyl, $-SC_6H_5$ oder $-SC(CH_3)_2CN$ steht;

wobei Säureadditionssalze sowie Metallkomplexe der Formel I mit eingeschlossen sind.

2. Mittel nach Anspruch 1, enthaltend mindestens ein Mandelsäurederivat der Formel I*

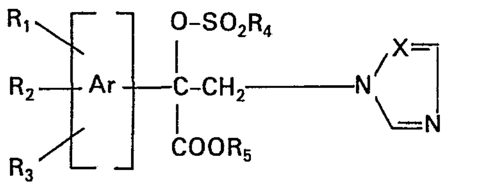

(I*),

worin

X für das Brückenglied -CH= oder -N= steht;

Ar eine Phenyl-, Diphenyl- oder Naphthylgruppe bedeutet;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen;

$R_5$ $C_1$-$C_4$-Alkyl, Phenyl, ein durch Nitro, Halogen und/oder Methyl ein- oder mehrfach substituiertes Phenyl oder Benzyl bedeutet;

$R_4$ für $C_1$-$C_6$-Alkyl, gegebenenfalls durch Fluor, Chlor, Brom, $CR_3$, Methyl, Methoxy oder Cyano substituiertes Phenyl oder ebenso gegebenenfalls substituiertes Benzyl steht oder die Gruppe $-N(R_9)(R_{10})$ repräsentiert; wobei

$R_9$ $C_1$-$C_3$-Alkyl bedeutet; und

$R_{10}$ für Wasserstoff, $C_1$-$C_3$-Alkyl, $-SCCl_3$, $-SCCl_2F$, $-SCCl_2CHCl_2$, $-SC_6H_5$ oder $-SC(CH_3)_2CN$ steht;

unter Einschluss der pflanzenverträglichen Säureadditionsalze sowie Metallkomplexe.

3. Mittel nach Anspruch 2, enthaltend mindestens ein Mandelsäurederivat der Formel I, worin X für das Brückenglied -CH= oder -N= steht; Ar eine Phenylgruppe bedeutet; $R_1$ in ortho-Position für Wasserstoff, Nitro, Halogen, $C_1$-$C_3$-Alkyl, $C_1$-$C_3$-Alkoxy oder $C_1$-$C_3$-Haloalkyl steht; $R_2$ in para-Position für Wasserstoff, Nitro, Halogen, $C_1$-$C_3$-Alkyl, $C_1$-$C_3$-Alkoxy oder $C_1$-$C_3$-Haloalkyl steht; $R_3$ Wasserstoff, Methyl oder Halogen bedeutet; R eine der Gruppen $-COOR_5$, $-COSR_6$,

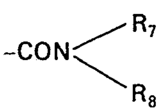

oder -CN darstellt; $R_5$ $C_1$-$C_4$-Alkyl, Phenyl, ein durch Nitro, Halogen und/oder Methyl ein- oder mehrfach substituiertes Phenyl oder ebenso substituiertes Benzyl bedeutet; $R_6$ $C_1$-$C_{10}$-Alkyl oder eine unsubstituierte oder durch Halogen, $C_1$-$C_4$-Alkyl, $C_1$-$C_4$-Alkoxy, -CN oder $-CF_3$ substituierte Phenyl- oder Benzylgruppe bedeutet, $R_7$ und $R_8$ unabhängig voneinander für Wasserstoff, $C_1$-$C_3$-Alkyl, $C_3$-$C_7$-Cycloalkyl, Phenyl oder Benzyl stehen; und $R_4$ für $C_1$-$C_4$-Alkyl, gegebenenfalls durch Fluor, Chlor, Brom, $CF_3$, Methyl oder Methoxy substituiertes Phenyl oder gegebenenfalls ebenso substituiertes Benzyl steht oder die Gruppe $-N(R_9)(R_{10})$ repräsentiert; wobei $R_9$ für $C_1$-$C_3$-Alkyl steht; und $R_{10}$ Wasserstoff, $C_1$-$C_3$-Alkyl, $-SCCl_3$, $-SCCl_2F$, $-SCCl_2CHCl_2$, $-SC_6H_5$ sowie Metallkomplexe der Formel I mit eingeschlossen sind.

4. Mittel nach Anspruch 2, enthaltend mindestens ein Mandelsäurederivat der Formel I, worin X für das Brückenglied -N= steht;

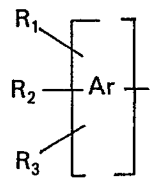

eine in ortho- und/oder para-Position durch Nitro, Fluor, Chlor, Brom, Methyl, Methoxy und/oder $CF_3$ substituierte Phenylgruppe bedeutet; R für die Gruppe $-COOR_5$ steht; $R_5$ $C_1$-$C_4$-Alkyl, Phenyl, ein durch Nitro, Chlor, Brom, Fluor und/oder Methyl substituiertes Phenyl oder ebenso substituiertes Benzyl bedeutet; und $R_4$ für Methyl, Ethyl, Phenyl, p-Toluol, p-Methylbenzyl, $-NH(C_1$-$C_4$-Alkyl) oder $-N(R_9)(R_{10})$ steht; wobei $R_9$ $C_1$-$C_2$-Alkyl und $R_{10}$ Methyl, $-SCCl_3$, $-SCCl_2F$, $-SCCl_2CHCl_2$, $-SC_6H_5$ oder $-SC(CH_3)_2CN$ bedeuten; wobei Säureadditionssalze sowie Metallkomplexe der Formel I mit eingeschlossen sind.

5. Mittel nach Anspruch 1, enthaltend mindestens eines der nachfolgenden Mandelsäurederivate der Formel I, nämlich

2-(1H-1,2,4-Triazolylmethyl-1′-yl)-2-methylsulfonyloxy-2-chlor-4-bromphenylessigsäureethylester;

2-(1H-1,2,4-Triazolylmethyl-1′-yl)-2-methylsulfonyloxy-2,4-dichlorphenylessigsäureethylester;

2-(1H-1,2,4-Triazolylmethyl-1′-yl)-2-(N-methyl-N-fluordichlormethansulfenyl-sulfamoyloxy)-2-chlor-4-bromphenylessigsäureethylester;

2-(1H-1,2,4-Triazolylmethyl-1′-yl)-2-(N-ethylsulfamoyloxy)-2,4-dichlorphenylessigsäuremethylester;

2-(1H-1,2,4-Triazolylmethyl-1′-yl)-2-(N-methylsulfamoyloxy)-2-chlor-4-bromphenylessigsäureethylester;

2-(1H-1,2,4-Triazolylmethyl-1′-yl)-2-(N,N-dimethylsulfamoyloxy)-2-chlor-4-bromphenylessigsäureethylester;

2-(1H-1,2,4-Triazolylmethyl-1′-yl)-2-methylsulfonyloxy-4-chlorphenylessigsäureethylester;

2-(1H-1,2,4-Triazolylmethyl-1′-yl)-2-(N-methylsulfonyloxy)-4-chlorphenylessigsäureethylester;

2-(1H-1,2,4-Triazolylmethyl-1′-yl)-2-(N-methyl-N-fluordichlormethansulfenyl-sulfamoyloxy)-4-chlorphenylessigsäureethylester;

2-(1H-1,2,4-Triazolylmethyl-1′-yl)-2-methylsulfonyloxy-2-chlor-4-bromphenylessigsäureisopropylester;

2-(1H-1,2,4-Triazolylmethyl-1′-yl)-2-ethylsulfonyloxy-2-chlor-4-bromphenylessigsäureethylester;

2-(1H-1,2,4-Triazolylmethyl-1′-yl)-2-chlormethylsulfonyloxy-2-chlor-4-bromphenylessigsäureethylester;

2-(1H-1,2,4-Triazolylmethyl-1′-yl)-2-paratosyloxy-2-chlor-4-bromphenylessigsäureethylester;

2-(1H-1,2,4-Triazolylmethyl-1′-yl)-2-(N-trichlormethansulfenylsulfamoyloxy)-2-chlor-4-bromphenylessigsäureethylester;

2-(1H-1,2,4-Triazolylmethyl-1′-yl)-2-methylsulfonyloxy-2,4-dichlorphenylessigsäuremethylester; und
2-(1H-1,2,4-Triazolylmethyl-1′-yl)-2-methylsulfonyloxy-2,4-dichlorphenylessigsäure-n-butylester.

6. Verfahren zur Herstellung der in Anspruch 1 definierten Mandelsäurederivate der Formel I, dadurch gekennzeichnet, dass man einen Alkohol der Formel II

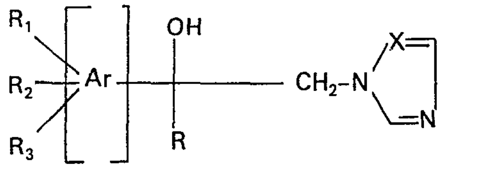
(II)

in An- oder Abwesenheit eines reaktionsinerten organischen Lösungs- oder Verdünnungsmittels, bei Temperaturen von −20°C bis 150°C entweder direkt in Gegenwart einer organischen oder anorganischen Base mit einem Sulfonylhalogenid der Formel XVI

$HalSO_2R_4$ (XVI)

an der OH-Gruppe sulfonyliert oder den Alkohol II vor der Sulfonylierung mit XVI zuerst in ein Alkalialkoholat der Formel III

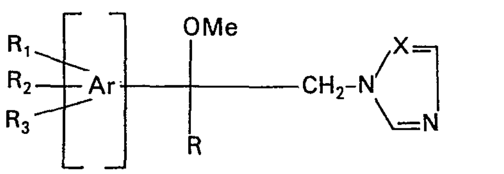
(III)

überführt, wobei die Substituenten in den Formeln II, III und XVI wie unter Formel I definiert sind, Hal für ein Halogenatom steht, und Me ein Alkalimetallatom bedeutet, und, falls erwünscht, die verfahrensgemäss erhältliche Verbindung der Formel I in eine andere Verbindung der Formel I umwandelt und/oder eine verfahrensgemäss erhältliche freie Verbindung in ein Säureadditionssalz umwandelt, ein verfahrensgemäss erhältliches Säureadditionssalz in die freie Verbindung oder ein anderes Säureadditionssalz umwandelt, oder eine verfahrensgemäss erhältliche Verbindung bzw. ein verfahrensgemäss erhältliches Salz in einen Metallkomplex überführt.

7. Verfahren nach Anspruch 6, dadurch gekennzeichnet, dass man die Umsetzung von II mit XVI bei Temperaturen von 0°C bis +40°C und von III mit XVI bei Temperaturen von 0°C bis +25°C durchführt, wobei der Substituent Hal in Formel XVI für Chlor oder Brom steht und Me in Formel III Natrium oder Kalium bedeutet.

8. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 5 auf die Pflanze oder deren Standort appliziert.

9. Verfahren zur Herstellung eines Schädlingsbekämpfungsmittels gemäss Anspruch 1, dadurch gekennzeichnet, dass mindestens eine Verbindung der Formel I mit geeigneten festen und/oder flüssigen Trägerstoffen und/oder Tensiden oder anderen applikationsfördernden Zusätzen innig vermischt wird und homogenisiert.

**Claims for the Contracting States: DE, GB, FR, CH, LI, IT, NL, BE, SE**

1. A mandelic acid derivative of the formula I

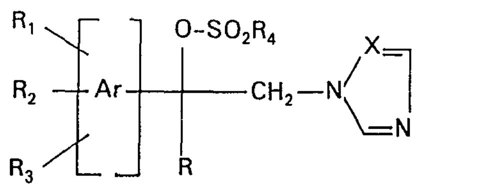
(I),

wherein

X is the bridge member -CH= or -N=,

Ar is a phenyl, diphenyl or naphthyl group,

$R_1$, $R_2$ and $R_3$ independently of one another are hydrogen, nitro, halogen, $C_1$-$C_3$alkyl, $C_1$-$C_3$alkoxy or $C_1$-$C_3$haloalkyl,

R is one of the groups, $-COOR_5$, $-COSR_6$,

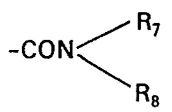

or -CN,

$R_5$ is $C_2$-$C_{10}$alkenyl which is unsubstituted or substituted by halogen; $C_2$-$C_{10}$alkynyl which is unsubstituted or substituted by halogen; or is a $C_3$-$C_8$cycloalkyl group or a phenyl group which is unsubstituted or substituted by halogen, $C_1$-$C_4$alkyl, $C_1$-$C_4$alkoxy, -CN or $-CF_3$; or is a $C_1$-$C_{12}$alkyl chain which from $C_2$alkyl may be interrupted by oxygen or sulfur and is unsubstituted or substituted by a member selected from the group consisting of halogen, phenyl, -COO-$C_1$-$C_4$alkyl, -CO-$C_1$-$C_4$alkyl, -CO-phenyl, an unsaturated or saturated 5- or 6membered ring containing oxygen or sulfur as heteroatom, with each phenyl moiety being unsubstituted or substituted by one or more identical or different halogen atoms,

$R_6$ is $C_1$-$C_{10}$alkyl, or is a phenyl or benzyl group, each unsubstituted or substituted by halogen, $C_1$-$C_4$alkyl, $C_1$-$C_4$alkoxy, -CN or $-CF_3$,

$R_7$ and $R_8$, each independently of the other, are hydrogen, $C_1$-$C_6$alkyl, $C_3$-$C_7$cycloalkyl, or a phenyl or benzyl group in each of which the aromatic ring is unsubstituted or substituted by halogen, $C_1$-$C_4$alkyl, $C_1$-$C_4$alkoxy, -CN or $-CF_3$, or both taken together form a 5- or 6membered saturated or unsaturated heterocyclic ring which may additionally contain 1 or 2 further N atoms,

$R_4$ is $C_1$-$C_{12}$alkyl which is unsubstituted or substituted by halogen or $C_1$-$C_4$alkoxy, or is phenyl or benzyl, each unsubstituted or substituted by halogen, $C_1$-$C_4$alkyl, $C_1$-$C_4$alkoxy, $C_1$-$C_4$haloalkyl

or cyano, or is the $-N(R_9)(R_{10})$ group, wherein $R_9$ is $C_1$–$C_6$alkyl and $R_{10}$ is hydrogen, $C_1$–$C_4$alkyl, $C_1$–$C_4$haloalkylsulfenyl, $-SC_6H_5$ or $-SC(CH_3)_2CN$, or an acid addition salt or metal complex thereof.

2. A mandelic acid derivative according to claim 1 of the formula I*

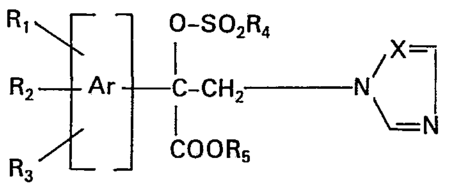

(I*),

wherein X is the bridge member -CH= or -N=, Ar is a phenyl, diphenyl or naphthyl group, $R_1$, $R_2$ and $R_3$, each independently of the other, are hydrogen, nitro, halogen, $C_1$–$C_3$alkyl, $C_1$–$C_3$-alkoxy or $C_1$–$C_3$haloalkyl, $R_5$ is $C_1$–$C_4$alkyl, phenyl, or phenyl or benzyl, each substituted by one or more nitro groups, halogen atoms and/or methyl groups, $R_4$ is $C_1$–$C_6$alkyl, or phenyl or benzyl, each unsubstituted or substituted by fluorine, chlorine, bromine, $CF_3$, methyl, methoxy or cyano, or is $-N(R_9)(R_{10})$, wherein $R_9$ is $C_1$–$C_3$alkyl, and $R_{10}$ is hydrogen, $C_1$–$C_3$alkyl, $-SCCl_3$, $-SCCl_2F$, $-SCCl_2CHCl_2$, $-SC_6H_5$ or $-SC(CH_3)_2CN$, or an agriculturally acceptable acid addition salt, quaternary azolium or ammonium salt or metal complex thereof.

3. A mandelic acid derivative of the formula I according to claim 2, wherein X is the bridge member -CH= or -N=; Ar is a phenyl group; $R_1$ in the ortho-position is hydrogen, nitro, halogen, $C_1$–$C_3$alkyl, $C_1$–$C_3$alkoxy, or $C_1$–$C_3$haloalkyl; $R_2$ in the para-position is hydrogen, nitro, halogen, $C_1$–$C_3$alkyl, $C_1$–$C_3$alkoxy or $C_1$–$C_3$haloalkyl; $R_3$ is hydrogen, methyl or halogen; R is a group $-COOR_5$, $-COSR_6$,

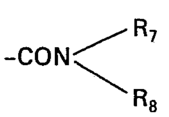

or -CN; $R_5$ is $C_1$–$C_4$alkyl, phenyl, or phenyl or benzyl, each substituted by one or more nitro groups, halogen atoms and/or methyl groups; $R_6$ is $C_1$–$C_{10}$alkyl, or phenyl or benzyl, each unsubstituted or substituted by halogen, $C_1$–$C_4$-alkyl, $C_1$–$C_4$alkoxy, -CN or $-CF_3$; each of $R_7$ and $R_8$ independently is hydrogen, $C_1$–$C_3$alkyl, $C_3$–$C_7$cycloalkyl, phenyl or benzyl; and $R_4$ is $C_1$–$C_4$alkyl, or is phenyl or benzyl, each unsubstituted or substituted by fluorine, chlorine, bromine, $CF_3$, methyl or methoxy, or is the $-N(R_9)(R_{10})$ group, wherein $R_9$ is $C_1$–$C_3$alkyl and $R_{10}$ is hydrogen, $C_1$–$C_3$alkyl, $-SCCl_3$, $-SCCl_2F$, $-SCCl_2CHCl_2$, $-SC_6H_5$ or $-SC(CH_3)_2CN$, or an acid addition salt or metal complex thereof.

4. A mandelic acid derivative of the formula I according to claim 2, wherein X is the bridge member -N=; the grouping

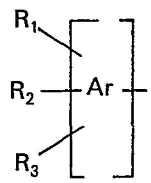

is a phenyl group which is substituted in the ortho- and/or para-position by nitro, fluorine, chlorine, bromine, methyl, methoxy and/or $CF_3$; R is the $-COOR_5$ group; $R_5$ is $C_1$–$C_4$ alkyl, phenyl, or phenyl or benzyl, each substituted by nitro, chlorine, bromine, fluorine and/or methyl; and $R_4$ is methyl, ethyl, phenyl, p-tolyl, p-methylbenzyl, $NH(C_1$–$C_4)$-alkyl or $-N(R_9)(R_{10})$, wherein $R_9$ is $C_1$–$C_2$alkyl and $R_{10}$ is methyl, $-SCCl_3$, $-SCCl_2F$, $-SCCl_2CHCl_2$, $-SC_6H_5$ or $-SC(CH_3)_2CN$, or an acid addition salt or metal complex thereof.

5. A mandelic acid derivative of the formula I selected from the group consisting of
ethyl 2-(1H-1,2,4-triazolylmethyl-1′-yl)-2-methylsulfonyloxy-2-chloro-4-bromophenylacetate,
ethyl 2-(1H-1,2,4-triazolylmethyl-1′-yl)-2-methylsulfonyloxy-2,4-dichlorophenylacetate,
ethyl 2-(1H-1,2,4-triazolylmethyl-1′-yl)-2-(N-methyl-N-fluorodichloromethanesulfenylsulfamoyloxy)-2-chloro-4-bromophenylacetate,
methyl 2-(1H-1,2,4-triazolylmethyl-1′-yl)-2-(N-ethylsulfamoyloxy)-2,4-dichlorophenylacetate,
ethyl 2-(1H-1,2,4-triazolylmethyl-1′-yl)-2-(N-methylsulfamoyloxy)-2-chloro-4-bromophenylacetate,
ethyl 2-(1H-1,2,4-triazolylmethyl-1′-yl)-2-(N,N-dimethylsulfamoyloxy)-2-chloro-4-bromophenylacetate,
ethyl 2-(1H-1,2,4-triazolylmethyl-1′-yl)-2-methylsulfonyloxy-4-chlorophenylacetate,
ethyl 2-(1H-1,2,4-triazolylmethyl-1′-yl)-2-(N-methylsulfonyloxy)-4-chlorophenylacetate,
ethyl 2-(1H-1,2,4-triazolylmethyl-1′-yl)-2-(N-methyl-N-fluorodichloromethanesulfenylsulfamoyloxy)-4-chlorophenylacetate,
isopropyl 2-(1H-1,2,4-triazolylmethyl-1′-yl)-2-methylsulfonyloxy-2-chloro-4-bromophenylacetate,
ethyl 2-(1H-1,2,4-triazolylmethyl-1′-yl)-2-ethylsulfonyloxy-2-chloro-4-bromophenylacetate,
ethyl 2-(1H-1,2,4-triazolylmethyl-1′-yl)-2-chloromethylsulfonyloxy-2-chloro-4-bromophenylacetate,
ethyl 2-(1H-1,2,4-triazolylmethyl-1′-yl)-2-p-tosyloxy-2-chloro-4-bromophenylacetate,
ethyl 2-(1H-1,2,4-triazolylmethyl-1′-yl)-2-(N-trichloromethanesulfenylsulfamoyloxy)-2-chloro-4-bromophenylacetate,
methyl 2-(1H-1,2,4-triazolylmethyl-1′-yl)-2-methylsulfonyloxy-2,4-dichlorophenylacetate,
and
n-butyl 2-(1H-1,2,4-triazolylmethyl-1′-yl)-2-methylsulfonyloxy-2,4-dichlorophenylacetate.

6. A process for the preparation of a mandelic acid derivative of the formula I as defined in claim

1, which process comprises sulfonylating an alcohol of the formula II

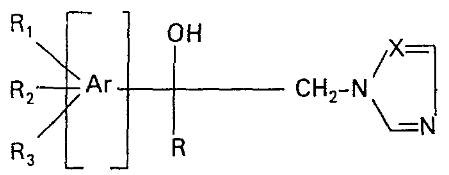

(II)

at the OH group with a sulfonyl halide of the formula XVI

$HalSO_2R_4$ (XVI)

in the absence or presence of an inert organic solvent or diluent, in the temperature range from −20°C to +150°C and in the presence of an organic or inorganic base, or converting the alcohol of formula II, before the sulfonylation with XVI, first into an alkali alcoholate of the formula III

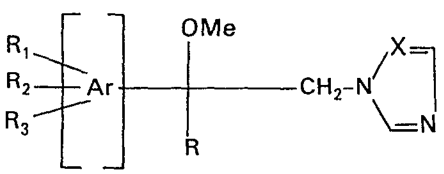

(III)

in which formulae II, III and XVI above, the substituents R, $R_1$, $R_2$, $R_3$, $R_4$, X and Ar are as defined for formula I, Hal is a halogen atom, and Me is an alkali metal atom, and, if desired, converting the resultant compound of the formula I into another compound of the formula I, and/or converting a free compound obtainable by the process into an acid addition salt, or converting an acid addition salt obtainable by the process into the free compound or into another acid addition salt, or converting a free compound or a salt obtainable by the process of the invention into a metal complex.

7. A process according to claim 6, wherein the reaction of the alcohol of formula II with the sulfonyl halide of formula XVI is carried out in the temperature range from 0°C to +40°C, and the reaction of the alcoholate of formula III with the sulfonyl halide of formula XVI is carried out in the temperature range from 0°C to +25°C, Hal in formula XVI being chlorine or bromine and Me in formula III being sodium or potassium.

8. A pesticidal composition for controlling microorganisms or for preventing infestation by such microorganisms, which composition contains as at least one active ingredient a compound of the formula I according to anyone of claims 1 to 5, together with carriers.

9. A method of controlling phytopathogenic microorganisms or of protecting cultivated plants from attack by such microorganisms, which method comprises applying to said plants or to the locus thereof a microbicidally effective amount of a compound of the formula I as claimed in anyone of claims 1 to 5.

10. A process for the preparation of a pesticidal composition according to claim 8, which process comprises homogeneously mixing at least one compound of the formula I according to claim 1 with suitable solid and/or liquid carriers and/or surfactants.

**Claims for the Contracting State: AT**

1. A pesticidal composition for controlling phytopathogenic microorganisms or for protecting plants from attack by such microorganisms, which composition contains as active ingredient at least one mandelic acid derivative of the formula I

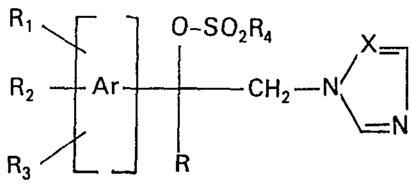

(I),

wherein

X is the bridge member -CH= or -N=,

Ar is a phenyl, diphenyl or naphthyl group,

$R_1$, $R_2$ and $R_3$ independently of one another are hydrogen, nitro, halogen, $C_1$-$C_3$alkyl, $C_1$-$C_3$alkoxy or $C_1$-$C_3$haloalkyl,

R is one of the groups $-COOR_5$, $-COSR_6$,

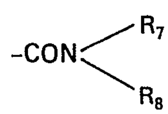

or -CN,

$R_5$ is $C_2$-$C_{10}$alkenyl which is unsubstituted or substituted by halogen; $C_2$-$C_{10}$alkynyl which is unsubstituted or substituted by halogen; or is a $C_3$-$C_8$ cycloalkyl group or a phenyl group which is unsubstituted or substituted by halogen, $C_1$-$C_4$alkyl, $C_1$-$C_4$alkoxy, -CN or $-CF_3$; or is a $C_1$-$C_{12}$alkyl chain which from $C_2$alkyl may be interrupted by oxygen or sulfur and is unsubstituted or substituted by a member selected from the group consisting of halogen, phenyl, -COO-$C_1$-$C_4$alkyl, -CO-$C_1$-$C_4$alkyl, -CO-phenyl, an unsaturated or saturated 5- or 6-membered ring containing oxygen or sulfur as heteroatom, which each phenyl moiety being unsubstituted or substituted by one or more identical or different halogen atoms,

$R_6$ is $C_1$-$C_{10}$alkyl, or is a phenyl or benzyl group, each unsubstituted or substituted by halogen, $C_1$-$C_4$alkyl $C_1$-$C_4$alkoxy, -CN or $-CF_3$,

$R_7$ and $R_8$, each independently of the other, are hydrogen, $C_1$-$C_6$alkyl, $C_3$-$C_7$cycloalkyl, or a phenyl or benzyl group in each of which the aromatic ring is unsubstituted or substituted by halogen, $C_1$-$C_4$ alkyl, $C_1$-$C_4$ alkoxy, -CN or $-CF_3$, or unsaturated or saturated 5- or 6membered ring saturated or unsaturated heterocyclic ring which may additionally contain 1 or 2 further N atoms,

$R_4$ is $C_1$-$C_{12}$ alkyl which is unsubstituted or sub-

stituted by halogen or $C_1$–$C_4$ alkoxy, or is phenyl or benzyl, each unsubstituted or substituted by halogen, $C_1$–$C_4$ alkyl, $C_1$–$C_4$ alkoxy, $C_1$–$C_4$ haloalkyl or cyano, or is the $-N(R_9)(R_{10})$ group, wherein

$R_9$ is $C_1$–$C_6$alkyl and

$R_{10}$ is hydrogen, $C_1$–$C_4$alkyl, $C_1$–$C_4$haloalkylsulfenyl, $-SC_6H_5$ or $-SC(CH_3)_2CN$,

or an acid addition salt or metal complex thereof, together with customary carrier materials and application promoters.

2. A composition according to claim 1, containing at least one mandelic acid derivative of the formula I*

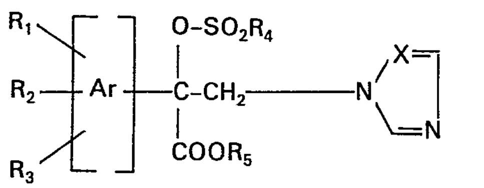
(I*),

wherein

X is the bridge member -CH= or -N=,

Ar is a phenyl, diphenyl or naphthyl group,

$R_1$, $R_2$ and $R_3$, each independently of the other, are hydrogen, nitro, halogen, $C_1$–$C_3$alkyl, $C_1$–$C_3$alkoxy or $C_1$–$C_3$haloalkyl,

$R_5$ is $C_1$–$C_4$alkyl, phenyl, or phenyl or benzyl, each substituted by one or more nitro groups, halogen atoms and/or methyl groups,

$R_4$ is $C_1$–$C_6$alkyl, or phenyl or benzyl, each unsubstituted or substituted by fluorine, chlorine, bromine, $CF_3$, methyl, methoxy or cyano, or is $-N(R_9)(R_{10})$, wherein

$R_9$ is $C_1$–$C_3$alkyl, and

$R_{10}$ is hydrogen, $C_1$–$C_3$alkyl, $-SCCl_3$, $-SCCl_2F$, $-SCCl_2CHCl_2$, $-SC_6H_5$ or $-SC(CH_3)_2CN$,

or an agriculturally acceptable acid addition salt or metal complex thereof.

3. A composition according to claim 2, containing at least one mandelic acid derivative of the formula I, wherein X is the bridge member -CH= or -N=; Ar is a phenyl group; $R_1$ in the ortho-position is hydrogen, nitro, halogen, $C_1$–$C_3$alkyl, $C_1$–$C_3$alkoxy, or $C_1$–$C_3$haloalkyl; $R_2$ in the para-position is hydrogen, nitro, halogen, $C_1$–$C_3$alkyl, $C_1$–$C_3$alkoxy or $C_1$–$C_3$haloalkyl; $R_3$ is hydrogen, methyl or halogen; R is a group $-COOR_5$, $-COSR_6$,

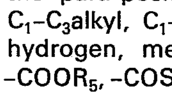

or -CN; $R_5$ is $C_1$–$C_4$alkyl, phenyl, or phenyl or benzyl, each substituted by one or more nitro groups, halogen atoms and/or methyl groups; $R_6$ is $C_1$–$C_{10}$alkyl, or phenyl or benzyl, each unsubstituted or substituted by halogen, $C_1$–$C_4$alkyl, $C_1$–$C_4$alkoxy, -CN or $-CF_3$; each of $R_7$ and $R_8$ independently is hydrogen, $C_1$–$C_3$alkyl, $C_3$–$C_7$cycloalkyl, phenyl or benzyl; and $R_4$ is $C_1$–$C_4$alkyl, or is phenyl or benzyl, each unsubstituted or substituted by fluorine, chlorine, bromine, $CF_3$, methyl or methoxy, or is the $-N(R_9)(R_{10})$ group, wherein $R_9$ is $C_1$–$C_3$alkyl and $R_{10}$ is hydrogen, $C_1$–$C_3$alkyl, $-SCCl_3$, $-SCCl_2F$, $-SCCl_2CHCl_2$, $-SC_6H_5$ or $-SC(CH_3)_2CN$, or an acid addition salt or metal complex thereof.

4. A composition according to claim 2, containing at least one mandelic acid derivative of the formula I, wherein X is the bridge member -N=; the grouping

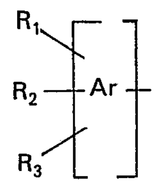

is a phenyl group which is substituted in the ortho- and/or para-position by nitro, fluorine, chlorine, bromine, methyl, methoxy and/or $CF_3$; R is the $-COOR_5$ group; $R_5$ is $C_1$–$C_4$ alkyl, phenyl, or phenyl or benzyl, each substituted by nitro, chlorine, bromine, fluorine and/or methyl; and $R_4$ is methyl, ethyl, phenyl, p-tolyl, p-methylbenzyl, $NH(C_1$–$C_4)$ alkyl or $-N(R_9)(R_{10})$, wherein $R_9$ is $C_1$–$C_2$ alkyl und $R_{10}$ is methyl, $-SCCl_3$, $-SCCl_2F$, $-SCCl_2CHCl_2$, $-SC_6H_5$ or $-SC(CH_3)_2CN$, or an acid addition salt or metal complex thereof.

5. A composition according to claim 1, containing at least one of the following mandelic acid derivatives of the formula I

ethyl 2-(1H-1,2,4-triazolylmethyl-1′-yl)-2-methylsulfonyloxy-2-chloro-4-bromophenylacetate,

ethyl 2-(1H-1,2,4-triazolylmethyl-1′-yl)-2-methylsulfonyloxy-2,4-dichlorophenylacetate,

ethyl 2-(1H-1,2,4-triazolylmethyl-1′-yl)-2-(N-methyl-N-fluorodichloromethanesulfenylsulfamoyloxy)-2-chloro-4-bromophenylacetate,

methyl 2-(1H-1,2,4-triazolylmethyl-1′-yl)-2-(N-ethylsulfamoyloxy)-2,4-dichlorophenylacetate,

ethyl 2-(1H-1,2,4-triazolylmethyl-1′-yl)-2-(N-methylsulfamoyloxy)-2-chloro-4-bromophenylacetate,

ethyl 2-(1H-1,2,4-triazolylmethyl-1′-yl)-2-(N,N-dimethylsulfamoyloxy)-2-chloro-4-bromophenylacetate,

ethyl 2-(1H-1,2,4-triazolylmethyl-1′-yl)-2-methylsulfonyloxy-4-chlorophenylacetate,

ethyl 2-(1H-1,2,4-triazolylmethyl-1′-yl)-2-(N-methylsulfonyloxy)-4-chlorophenylacetate,

ethyl 2-(1H-1,2,4-triazolylmethyl-1′-yl)-2-(N-methyl-N-fluorodichloromethanesulfenylsulfamoyloxy)-4-chlorophenylacetate,

isopropyl 2-(1H-1,2,4-triazolylmethyl-1′-yl)-2-methylsulfonyloxy-2-chloro-4-bromophenylacetate,

ethyl 2-(1H-1,2,4-triazolylmethyl-1′-yl)-2-ethylsulfonyloxy-2-chloro-4-bromophenylacetate,

ethyl 2-(1H-1,2,4-triazolylmethyl-1′-yl)-2-chloromethylsulfonyloxy-2-chloro-4-bromophenylacetate,

ethyl 2-(1H-1,2,4-triazolylmethyl-1′-yl)-2-p-tosyloxy-2-chloro-4-bromophenylacetate,

ethyl 2-(1H-1,2,4-triazolylmethyl-1′-yl)-2-(N-trichloromethanesulfenylsulfamoyloxy)-2-chloro-4-bromophenylacetate,

methyl 2-(1H-1,2,4-triazolylmethyl-1′-yl)-2-methylsulfonyloxy-2,4-dichlorophenylacetate, and
n-butyl 2-(1H-1,2,4-triazolylmethyl-1′-yl)-2-methylsulfonyloxy-2,4-dichlorophenylacetate.

6. A process for the preparation of a mandelic acid derivative of the formula I as defined in claim 1, which process comprises sulfonylating an alcohol of the formula II

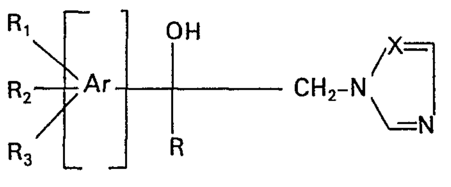

(II)

at the OH group with a sulfonyl halide of the formula XVI

$HalSO_2R_4$ (XVI)

in the absence or presence of an inert organic solvent or diluent, in the temperature range from −20°C to +150°C and in the presence of an organic or inorganic base, or converting the alcohol of formula II, before the sulfonylation with XVI, first into an alkali alcoholate of the formula III

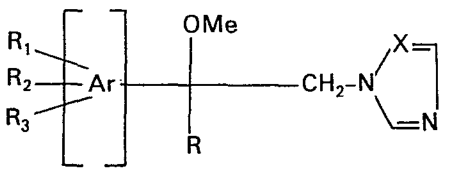

(III)

in which formulae II, III and XVI above, the substituents R, $R_1$, $R_2$, $R_3$, $R_4$, X and Ar are as defined for formula I, Hal is a halogen atom, and Me is an alkali metal atom, and, if desired, converting the resultant compound of the formula I into another compound of the formula I, and/or converting a free compound obtainable by the process into an acid addition salt, or converting an acid addition salt obtainable by the process into the free compound or into another acid addition salt, or converting a free compound or a salt obtainable by the process of the invention into a metal complex.

7. A process according to claim 6, wherein the reaction of the alcohol of formula II with the sulfonyl halide of formula XVI is carried out in the temperature range from 0° to +40°C, and the reaction of the alcoholate of formula III with the sulfonyl halide of formula XVI is carried out in the temperature range from 0° to + 25°C, Hal in formula XVI being chlorine or bromine and Me in formula III being sodium or potassium.

8. A method of controlling phytopathogenic microorganisms or of preventing infestation by such microorganisms, which comprises applying to said plants or to the locus thereof a microbicidally effective amount of a compound of the formula I as defined in any one of claims 1 to 5.

9. A process for the preparation of a pesticidal composition according to claim 1, which process comprises homogeneously mixing at least one compound of the formula I as defined in claim 1 with suitable solid and/or liquid carriers and/or surfactants or other application promoters.

**Revendications pour les Etats Contractants: BE, CH, DE, FR, GB, IT, LI, NL, SE**

1. Dérivés de l'acide mandélique de formule I

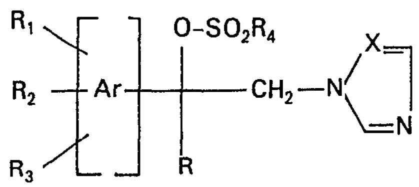

(I),

dans laquelle

X représente un pont -CH= ou -N=;

Ar représente un groupe phényle, diphényle ou naphtyle, $R_1$, $R_2$, $R_3$ représentent chacun, indépendamment les uns des autres, l'hydrogène, un groupe nitro, un halogène, un groupe alkyle en $C_1$-$C_3$, alcoxy en $C_1$-$C_3$ ou halogénoalkyle en $C_1$-$C_3$,

R représente l'un des groupes $-COOR_5$, $-COSR_6$,

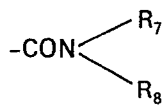

$R_5$ représente un groupe alcényle en $C_2$-$C_{10}$ non substitué ou substitué par des halogènes; un groupe alcynyle en $C_2$-$C_{10}$ non substitué ou substitué par des halogènes; ou un groupe cycloalkyle en $C_3$-$C_8$ ou un groupe phényle non substitué ou substitué par des halogènes, des groupes alkyle en $C_1$-$C_4$, alcoxy en $C_1$-$C_4$, -CN ou $-CF_3$; ou bien $R_5$ représente une chaîne alkyle en $C_1$-$C_{12}$ qui, à partir d'une chaîne alkyle en $C_2$, peut être interrompue par l'oxygène ou le soufre et qui est non substituée ou peut être substituée par l'un des atomes ou groupes suivants: halogènes, phényle, -COOalkyle en $C_1$-$C_4$, COalkyle en $C_1$-$C_4$, -COphényle, un cycle insaturé ou saturé à 5 ou 6 chaînons contenant l'oxygène ou le soufre en tant qu'hétéroatome, chaque groupe phényle étant non substitué ou mono- ou poly-substitué par des atomes d'halogènes identiques ou différents;

$R_6$ représente un groupe alkyle en $C_1$-$C_{10}$ ou un groupe phényle ou benzyle non substitué ou substitué par des halogènes, des groupes alkyle en $C_1$-$C_4$, alcoxy en $C_1$-$C_4$, -CN ou $-CF_3$;

$R_7$ et $R_8$ représentent chacun, indépendamment l'un de l'autre, l'hydrogène, un groupe alkyle en $C_1$-$C_6$, cycloalkyle en $C_3$-$C_7$ ou un groupe phényle ou benzyle dans chacun desquels le noyau aromatique peut être non substitué ou substitué par des halogènes, des groupes alkyle en $C_1$-$C_4$, alcoxy en $C_1$-$C_4$, -CN ou $-CF_3$, ou bien

$R_7$ et $R_8$ forment ensemble un noyau hétérocyclique saturé ou insaturé à 5 ou 6 chaînons qui peut encore contenir un ou deux autres atomes d'azote;

$R_4$ représente un groupe alkyle en $C_1$–$C_{12}$ éventuellement substitué par des halogènes ou un groupe alcoxy en $C_1$–$C_4$, un groupe phényle éventuellement substitué par des halogènes, des groupes alkyle en $C_1$–$C_4$, alcoxy en $C_1$–$C_4$, halogénoalkyle en $C_1$–$C_4$ ou cyano ou un groupe benzyle portant éventuellement les mêmes substituants, ou le groupe $-N(R_9)(R_{10})$ dans lequel

$R_9$ représente un groupe alkyle en $C_1$–$C_6$ et

$R_{10}$ représente l'hydrogène, un groupe alkyle en $C_1$–$C_4$, halogénoalkylsulfényle en $C_1$–$C_4$, $-SC_6H_5$ ou $-SC(CH_3)_2CN$;

y compris les sels formés par addition avec des acides et complexes métalliques de formule I.

2. Dérivés de l'acide mandélique selon la revendication 1, de formule I*

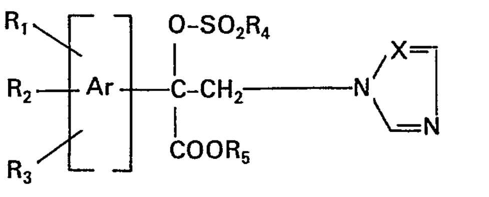

(I*),

dans laquelle

X représente le pont $-CH=$ ou $-N=$;

Ar représente un groupe phényle, diphényle ou naphtyle;

$R_1$, $R_2$, $R_3$ représentent chacun, indépendamment les uns des autres, l'hydrogène, un groupe nitro, un halogène, un groupe alkyle en $C_1$–$C_3$, alcoxy en $C_1$–$C_3$ ou halogénoalkyle en $C_1$–$C_3$;

$R_5$ représente un groupe alkyle en $C_1$–$C_4$, phényle, un groupe phényle ou benzyle mono- ou poly-substitué par des groupes nitro, des halogènes et/ou des groupes méthyle;

$R_4$ représente un groupe alkyle en $C_1$–$C_6$, un groupe phényle éventuellement substitué par le fluor, le chlore, le brome, des groupes $CF_3$, méthyle, méthoxy ou cyano ou un groupe benzyle portant éventuellement les mêmes substituants, ou le groupe $-N(R_9)(R_{10})$ dans lequel

$R_9$ représente un groupe alkyle en $C_1$–$C_3$, et

$R_{10}$ représente l'hydrogène, un groupe alkyle en $C_1$–$C_3$, $-SCCl_3$, $-SCCl_2F$, $SCCl_2CHCl_2$, $-SC_6H_5$ ou $-SC(CH_3)_2CN$; y compris les sels formés par addition avec des acides, sels d'azolium et d'ammonium quaternaire et complexes métalliques tolérés par les végétaux.

3. Dérivés de l'acide mandélique de formule I selon la revendication 2, dans laquelle X représente le pont $-CH=$ ou $-N=$; Ar représente un groupe phényle; $R_1$ représente, en position ortho, l'hydrogène, un groupe nitro, un halogène, un groupe alkyle en $C_1$–$C_3$, alcoxy en $C_1$–$C_3$ ou halogénoalkyle en $C_1$–$C_3$; $R_2$ représente, en position para, l'hydrogène, un groupe nitro, un halogène, un groupe alkyle en $C_1$–$C_3$, alcoxy en $C_1$–$C_3$ ou halogénoalkyle en $C_1$–$C_3$; $R_3$ représente l'hydrogène, un groupe méthyle ou un halogène; R représente l'un des groupes $-COOR_5$, $-COSR_6$,

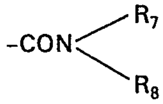

ou $-CN$; $R_5$ représente un groupe alkyle en $C_1$–$C_4$, phényle, un groupe phényle mono- ou poly-substitué par des groupes nitro, des halogènes et/ou des groupes méthyle, ou un groupe benzyle portant les mêmes substituants; $R_6$ représente un groupe alkyle en $C_1$–$C_{10}$ ou un groupe phényle ou benzyle non substitué ou substitué par des halogènes, des groupes alkyle en $C_1$–$C_4$, alcoxy en $C_1$–$C_4$, $-CN$ ou $-CF_3$, $R_7$ et $R_8$ représentent chacun, indépendamment l'un de l'autre, l'hydrogène, un groupe alkyle en $C_1$–$C_3$, cycloalkyle en $C_3$–$C_7$, phényle ou benzyle; et $R_4$ représente un groupe alkyle en $C_1$–$C_4$, phényle éventuellement substitué par le fluor, le chlor, le brome, des groupes $CF_3$, méthyle ou méthoxy ou un groupe benzyle portant éventuellement les mêmes substituants, ou le groupe $-N(R_9)(R_{10})$ dans lequel $R_9$ représente un groupe alkyle en $C_1$–$C_3$ et $R_{10}$ représente l'hydrogène, un groupe alkyle en $C_1$–$C_3$, $-SCCl_3$, $-SCCl_2F$, $-SCCl_2CHCl_2$, $-SC_6H_5$ ou $-SC(CH_3)_2CN$; y compris les sels formés par addition avec des acides et complexes métalliques de formule I.

4. Dérivés de l'acide mandélique de formule I selon la revendication 2, dans lesquels X représente le pont $-N=$;

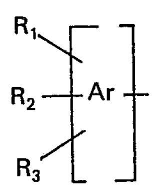

représente un groupe phényle substitué en position ortho et/ou para par des groupes nitro, le fluor, le chlore, le brome, des groupes méthyle, méthoxy et/ou $CF_3$; R représente le groupe $-COOR_5$; $R_5$ représente un groupe alkyle en $C_1$–$C_4$, phényle, un groupe phényle substitué par des groupes nitro, le chlore, le brome, le fluor et/ou des groupes méthyle, ou un groupe benzyle portant les mêmes substituants; et $R_4$ représente un groupe méthyle, éthyle, phényle, p-tolyle, p-méthylbenzyle, $-NH$(alkyle en $C_1$–$C_4$) ou $-N(R_9)(R_{10})$ dans lequel $R_9$ représente un groupe alkyle en $C_1$–$C_2$ et $R_{10}$ représente un groupe méthyle, $-SCCl_3$, $-SCCl_2F$, $-SCCl_2CHCl_2$, $-SC_6H_5$ ou $-SC(CH_3)_2CN$; y compris les sels formés par addition avec des acides et complexes métalliques de formule I.

5. Un dérivé de l'acide mandélique de formule I, choisi dans le groupe suivant:

2-(1H-1,2,4-triazolylméthyl-1'-yl)-2-méthylsulfonyloxy-2-chloro-4-bromophénylacétate d'éthyle;

2-(1H-1,2,4-triazolylméthyl-1'-yl)-2-méthylsulfoxyloxy-2,4-dichlorophénylacétate d'éthyle;

2-(1H-1,2,4-triazolylméthyl-1'-yl)-2-(N-méthyl-N-fluorodichlorométhane-sulfénylsulfamoyloxy)-2-chloro-4-bromophénylacétate d'éthyle;

2-(1H-1,2,4-triazolylméthyl-1'-yl)-2-(N-éthylsul-

famoyloxy)-2,4-dichlorophénylacétate de méthyle;
2-(1H-1,2,4-triazolylméthyl-1′-yl)-2-(N-méthylsulfamoyloxy)-2-chloro-4-bromophénylacétate d'éthyle;
2-(1H-1,2,4-triazolylméthyl-1′-yl)-2-(N,N-diméthylsulfamoyloxy)-2-chloro-4-bromophénylacétate d'éthyle;
2-(1H-1,2,4-triazolylméthyl-1′-yl)-2-méthylsulfonyloxy-4-chlorophénylacétate d'éthyle;
2-(1H-1,2,4-triazolylméthyl-1′-yl)-2-(N-méthylsulfonyloxy)-4-chloro-phénylacétate d'éthyle;
2-(1H-1,2,4-triazolylméthyl-1′-yl)-2-(N-méthyl-N-fluorodichlorométhane-sulfénylsulfamoyloxy)-4-chlorophénylacétate d'éthyle;
2-(1H-1,2,4-triazolylméthyl-1′-yl)-2-méthylsulfonyloxy-2-chloro-4-bromophénylacétate d'isopropyle;
2-(1H-1,2,4-triazolylméthyl-1′-yl)-2-éthylsulfonyloxy-2-chloro-4-bromophénylacétate d'éthyle;
2-(1H-1,2,4-triazolylméthyl-1′-yl)-2-chlorométhylsulfonyloxy-2-chloro-4-bromophénylacétate d'éthyle;
2-(1H-1,2,4-triazolylméthyl-1′-yl)-2-paratoluènesulfonyloxy-2-chloro-4-bromophénylacétate d'éthyle;
2-(1H-1,2,4-triazolylméthyl-1′-yl)-2-(N-trichlorométhane-sulfényl-sulfamoyloxy)-2-chloro-4-bromophénylacétate d'éthyle;
2-(1H-1,2,4-triazolylméthyl-1′-yl)-2-méthylsulfonyloxy-2,4-dichloro-phénylacétate de méthyle; et
2-(1H-1,2,4-triazolylméthyl-1′-yl)-2-méthylsulfonyloxy-2,4-dichlorophénylacétate de n-butyle.

6. Procédé de préparation des dérivés de l'acide mandélique de formule I définis dans la revendication 1, caractérisé en ce que l'on sulfonyle sur le groupe OH un alcool de formule II

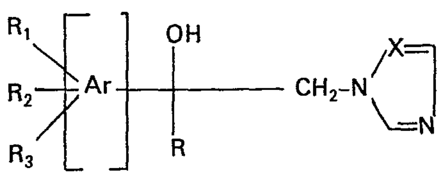
(II)

en présence ou en l'absence d'un solvant ou diluant organique inerte dans la réaction, à des températures de −20 à 150°C, soit directement, en présence d'une base organique ou minérale, à l'aide d'un halogénure de sulfonyle de formule XVI

$HalSO_2R_4$ (XVI)

soit après conversion de l'alcool II, avant la sulfonation par l'halogénure de sulfonyle XVI, en un alcoolate alcalin de formule III

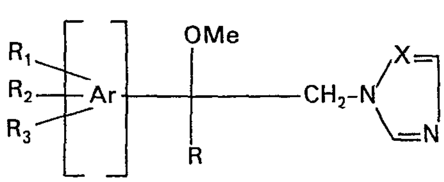
(III)

les symboles des formules II, III et XVI ayant été définis en référence à la formule I, Hal représente un atome d'halogène et Me un atome de métal alcalin, et si on le désire, on convertit un composé de formule I ainsi obtenu en un autre composé de formule I et/ou un composé libre obtenu en un sel formé par addition avec un acide, un sel d'acide obtenu en le composé libre ou en un autre sel d'acide, ou bien un composé ou un sel ainsi obtenu en un complexe métallique.

7. Procédé selon la revendication 6, caractérisé en ce que la réaction entre II et XVI est effectuée à des températures de 0 à +40°C et la réaction entre III et XVI à des températures de 0 à +5°C, le symbole Hal de la formule XVI représentant le chlore ou le brome et Me de la formule III le sodium ou le potassium.

8. Produit pesticide pour combattre ou prévenir une infestation par des microorganismes, caractérisés en ce qu'il contient au moins un composant actif consistant en un composé de formule I selon l'une des revendications 1 à 5, avec des véhicules.

9. Procédé pour combattre ou prévenir une infestation de végétaux cultivés par des microorganismes phytopathogènes, caractérisé en ce que l'on applique un composé de formule 1 selon l'une des revendications 1 à 5 sur les végétaux ou leur habitat.

10. Procédé de préparation d'un produit pesticide selon la revendication 6, caractérisé en ce que l'on mélange intimement au moins un composé de formule I selon la revendication 1 avec des véhicules solides et/ou liquides appropriés et/ou des agents tensioactifs.

**Revendications pour l'Etat contractant: AT**

1. Produit pesticide pour combattre ou prévenir une infestation de végétaux par des microorganismes phytopathogènes, caractérisé en ce qu'il contient, avec des véhicules usuels et des additifs facilitant l'application, au moins un composant actif consistant en un dérivé de l'acide mandélique de formule

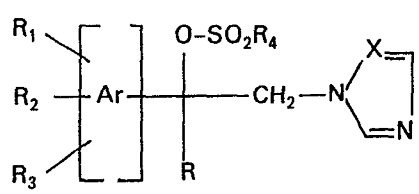
(I),

dans laquelle

X représente le pont -CH= ou -N=;

Ar représente un groupe phényle, diphényle ou naphtyle;

$R_1$, $R_2$, $R_3$ représentent chacun, indépendamment les uns des autres, l'hydrogène, un groupe nitro, un halogène, un groupe alkyle en $C_1$-$C_3$, alcoxy en $C_1$-$C_3$ ou halogénoalkyle en $C_1$-$C_3$,

R représente l'un des groupes $-COOR_5$, $-COSR_6$,

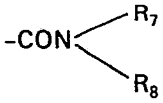

ou -CN,

$R_5$ représente un groupe alcényle en $C_2$-$C_{10}$ non substitué ou substitué par des halogènes; un groupe alcynyle en $C_2$-$C_{10}$ non substitué ou substitué par des halogènes; ou un groupe cycloalkyle en $C_3$-$C_8$ ou un groupe phényle non substitué ou substitué par des halogènes, des groupes alkyle en $C_1$-$C_4$, alcoxy en $C_1$-$C_4$, -CN ou -$CF_3$; ou bien $R_5$ représente une chaîne alkyle en $C_1$-$C_{12}$ qui, à partir d'une chaîne alkyle en $C_2$, peut être interrompue par l'oxygène ou le soufre et qui est non substitué ou peut être substitué par l'un des atomes ou groupes suivants: halogènes, phényle, -COOalkyle en $C_1$-$C_4$, -COalkyle en $C_1$-$C_4$, -COphényle, un cycle insaturé ou saturé à 5 ou 6 chaînons contenant l'oxygène ou le soufre en tant qu'hétéroatome, chaque groupe phényle étant non substitué ou mono- ou poly-substitué par des atomes d'halogènes identiques ou différents;

$R_6$ représente un groupe alkyle en $C_1$-$C_{10}$ ou un groupe phényle ou benzyle non substitué ou substitué par des halogènes, des groupes alkyle en $C_1$-$C_4$, alcoxy en $C_1$-$C_4$, -CN ou -$CF_3$;

$R_7$ et $R_8$ représentent chacun, indépendamment l'un de l'autre, l'hydrogène, un groupe alkyle en $C_1$-$C_6$, cycloalkyle en $C_3$-$C_7$ ou un groupe phényle ou benzyle dans chacun desquels le noyau aromatique peut être non substitué ou substitué par des halogènes, des groupes alkyle en $C_1$-$C_4$, alcoxy en $C_1$-$C_4$, -CN ou -$CF_3$, ou bien $R_7$ et $R_8$ forment ensemble un noyau hétérocyclique saturé ou insaturé à 5 ou 6 chaînons qui peut encore contenir un ou deux autres atomes d'azote;

$R_4$ représente un groupe alkyle en $C_1$-$C_{12}$ éventuellement substitué par des halogènes ou un groupe alcoxy en $C_1$-$C_4$, un groupe phényle éventuellement substitué par des halogènes, des groupes alkyle en $C_1$-$C_4$, alcoxy en $C_1$-$C_4$, halogénoalkyle en $C_1$-$C_4$ ou cyano ou un groupe benzyle portant éventuellement les mêmes substituants, ou le groupe -$N(R_9)(R_{10})$ dans lequel

$R_9$ représente un groupe alkyle en $C_1$-$C_6$ et

$R_{10}$ représente l'hydrogène, un groupe alkyle en $C_1$-$C_4$, halogénoalkylsulfényle en $C_1$-$C_4$, -$SC_6H_5$ ou -$SC(CH_3)_2CN$;

y compris les sels formés par addition avec des acides et complexes métalliques de formule I.

2. Produit selon la revendication 1, contenant au moins un dérivé de l'acide mandélique de formule I*

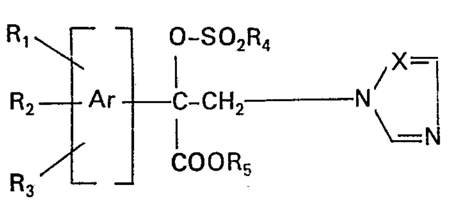

(I*),

dans laquelle

X représente le pont -CH= ou -N=;

Ar représente un groupe phényle, diphényle ou naphtyle;

$R_1$, $R_2$, $R_3$ représentent chacun, indépendamment les uns des autres, l'hydrogène, un groupe nitro, un halogène, un groupe alkyle en $C_1$-$C_3$, alcoxy en $C_1$-$C_3$ ou halogénoalkyle en $C_1$-$C_3$;

$R_5$ représente un groupe alkyle en $C_1$-$C_4$, phényle, un groupe phényle ou benzyle mono- ou poly-substitué par des groupes nitro, des halogènes et/ou des groupes méthyle;

$R_4$ représente un groupe alkyle en $C_1$-$C_6$, un groupe phényle éventuellement substitué par le fluor, le chlore, le brome, des groupes $CF_3$, méthyle, méthoxy ou cyano ou un groupe benzyle portant éventuellement les mêmes substituants, ou le groupe -$N(R_9)(R_{10})$ dans lequel

$R_9$ représente un groupe alkyle en $C_1$-$C_3$, et

$R_{10}$ représente l'hydrogène, un groupe alkyle en $C_1$-$C_3$, -$SCCl_3$, -$SCCl_2F$, $SCCl_2CHCl_2$, -$SC_6H_5$ ou -$SC(CH_3)_2CN$;

y compris les sels formés par addition avec des acides, sels d'azolium et d'ammonium quaternaire et complexes métalliques tolérés par les végétaux.

3. Produit selon la revendication 2, contenant au moins un dérivé de l'acide mandélique de formule I dans laquelle X représente le pont -CH= ou -N=; Ar représente un groupe phényle; $R_1$ représente, en position ortho, l'hydrogène, un groupe nitro, un halogène, un groupe alkyle en $C_1$-$C_3$, alcoxy en $C_1$-$C_3$ ou halogénoalkyle en $C_1$-$C_3$; $R_2$ représente, en position para, l'hydrogène, un groupe nitro, un halogène, un groupe alkyle en $C_1$-$C_3$, alcoxy en $C_1$-$C_3$ ou halogénoalkyle en $C_1$-$C_3$; $R_3$ représente l'hydrogène, un groupe méthyle ou un halogène; R représente l'un des groupes -$COOR_5$, -$COSR_6$,

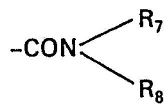

ou -CN; $R_5$ représente un groupe alkyle en $C_1$-$C_4$, phényle, un groupe phényle mono- ou poly-substitué par des groupes nitro, des halogènes et/ou des groupes méthyle, ou un groupe benzyle portant les mêmes substituants; $R_6$ représente un groupe alkyle en $C_1$-$C_{10}$ ou un groupe phényle ou benzyle non substitué ou substitué par des halogènes, des groupes alkyle en $C_1$-$C_4$, alcoxy en $C_1$-$C_4$, -CN ou -$CF_3$, $R_7$ ou $R_8$ représentent chacun, indépendamment l'un de l'autre, l'hydrogène, un groupe alkyle en $C_1$-$C_3$, cycloalkyle en $C_3$-$C_7$, phényle ou benzyle; et $R_4$ représente un groupe alkyle en $C_1$-$C_4$, phényle éventuellement substitué par le fluor, le chlore, le brome, des groupes $CF_3$, méthyle ou méthoxy ou un groupe benzyle portant éventuellement les mêmes substituants, ou le groupe -$N(R_9)(R_{10})$ dans lequel $R_9$ représente un groupe alkyle en $C_1$-$C_3$ et $R_{10}$ représente l'hydrogène, un groupe alkyle en $C_1$-$C_3$, -$SCCl_3$, -$SCCl_2F$, -$SCCl_2CHCl_2$, -$SC_6H_5$ ou -$SC(CH_3)_2CN$;

y compris les sels formés par addition avec des acides et complexes métalliques de formule I.

4. Produit selon la revendication 2, contenant au moins un dérivé de l'acide mandélique de formule I dans laquelle X représente le pont -N=;

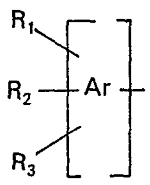

représente un groupe phényle substitué en position ortho et/ou para par des groupes nitro, le fluor, le chlore, le brome, des groupes méthyle, méthoxy et/ou $CF_3$; R représente le groupe $-COOR_5$; $R_5$ représente un groupe alkyle en $C_1$–$C_4$, phényle, un groupe phényle substitué par des groupes nitro, le chlore, le brome, le fluor et/ou des groupes méthyle, ou un groupe benzyle portant les mêmes substituants; et $R_4$ représente un groupe méthyle, éthyle, phényle, p-tolyle, p-méthylbenzyle, -NH(alkyle en $C_1$–$C_4$) ou $-N(R_9)(R_{10})$ dans lequel $R_9$ représente un groupe alkyle en $C_1$–$C_2$ et $R_{10}$ représente un groupe methyle, $-SCCl_3$, $-SCCl_2F$, $-SCCl_2CHCl_2$, $-SC_6H_5$ ou $-SC(CH_3)_2CN$; y compris les sels formés par addition avec des acides et complexes métalliques de formule I.

5. Produit selon la revendication 1, contenant au moins l'un des dérivés de l'acide mandélique de formule I ci-dessous, à savoir:

2-(1H-1,2,4-triazolylméthyl-1'-yl)-2-méthylsulfonyloxy-2-chloro-4-bromophénylacétate d'éthyle;
2-(1H-1,2,4-triazolylméthyl-1'-yl)-2-méthylsulfoxyloxy-2,4-dichlorophénylacétate d'éthyle;
2-(1H-1,2,4-triazolylméthyl-1'-yl)-2-(N-méthyl-N-fluorodichlorométhane-sulfénylsulfamoyloxy-2-chloro-4-bromophénylacétate d'éthyle;
2-(1H-1,2,4-triazolylméthyl-1'-yl)-2-(N-éthylsulfamoyloxy)-2,4-dichlorophénylacétate de méthyle;
2-(1H-1,2,4-triazolylméthyl-1'-yl)-2-(N-méthylsulfamoyloxy)-2-chloro-4-bromophénylacétate d'éthyle;
2-(1H-1,2,4-triazolylméthyl-1'-yl)-2-(N,N-diméthylsulfamoyloxy)-2-chloro-4-bromophénylacétate d'éthyle;
2-(1H-1,2,4-triazolylméthyl-1'-yl)-2-méthylsulfonyloxy-4-chlorophénylacétate d'éthyle;
2-(1H-1,2,4-triazolylméthyl-1'-yl)-2-(N-méthylsulfonyloxy)-4-chloro-phénylacétate d'éthyle;
2-(1H-1,2,4-triazolylméthyl-1'-yl)-2-(N-méthyl-N-fluorodichlorométhane-sulfénylsulfamoyloxy)-4-chlorophénylacétate d'éthyle;
2-(1H-1,2,4-triazolylméthyl-1'-yl)-2-méthylsulfonyloxy-2-chloro-4-bromophénylacétate d'isopropyle;
2-(1H-1,2,4-triazolylméthyl-1'-yl)-2-éthylsulfonyloxy-2-chloro-4-bromophénylacétate d'éthyle;
2-(1H-1,2,4-triazolylméthyl-1'-yl)-2-chlorométhylsulfonyloxy-2-chloro-4-bromophénylacétate d'éthyle;
2-(1H-1,2,4-triazolylméthyl-1'-yl)-2-paratoluènesulfonyloxy-2-chloro-4-bromophénylacétate d'éthyle;
2-(1H-1,2,4-triazolylméthyl-1'-yl)-2-(N-trichlorométhane-sulfényl-sulfamoyloxy)-2-chloro-4-bromophénylacétate d'éthyle;
2-(1H-1,2,4-triazolylméthyl-1'-yl)-2-méthylsulfonyloxy-2,4-dichloro-phénylacétate de méthyle; et
2-(1H-1,2,4-triazolylméthyl-1'-yl)-2-méthylsulfonyloxy-2,4-dichlorophénylacétate de n-butyle.

6. Procédé de préparation des dérivés de l'acide mandélique de formule I définis dans la revendication 1, caractérisé en ce que l'on sulfonyle sur le groupe OH un alcool de formule II

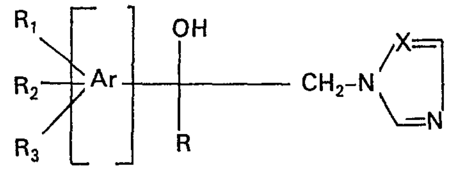

(II)

en présence ou en l'absence d'un solvant ou diluant organique inerte dans la réaction, à des températures de −20 à 150°C, soit directement, en présence d'une base organique ou minérale, à l'aide d'un halogénure de sulfonyle de formule XVI

$HalSO_2R_4$ (XVI)

soit après conversion de l'alcool II, avant la sulfonation par l'halogénure de sulfonyle XVI, en un alcoolate alcalin de formule III

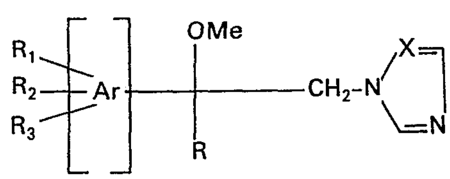

(III)

les symboles des formules II, III et XVI ayant été définis en référence à la formule I, Hal représente un atome d'halogène et Me un atome de métal alcalin, et si on le désire, on convertit un composé de formule I ainsi obtenu en un autre composé de formule I et/ou un composé libre obtenu en un sel formé par addition avec un acide, un sel d'acide obtenu en le composé libre ou en un autre sel d'acide, ou bien un composé ou un sel ainsi obtenu en un complexe métallique.

7. Procédé selon la revendication 6, caractérisé en ce que la réaction entre II et XVI est effectuée à des températures de 0 à +40°C et la réaction entre III et XVI à des températures de 0 à +5°C, le symbole Hal de la formule XVI représentant le chlore ou le brome et Me de la formule III le sodium ou le potassium.

8. Procédé pour combattre ou prévenir une infestation de végétaux cultivés par des microorganismes phytopathogènes, caractérisé en ce que l'on applique un composé de formule I selon l'une des revendications 1 à 5 sur les végétaux ou leur habitat.

9. Procédé de préparation d'un produit pesticide selon la revendication 1, caractérisé en ce que l'on mélange intimement et on homogénéise au moins un composé de formule I avec des véhicules solides et/ou liquides appropriés et/ou des agents tensioactifs ou d'autres additifs facilitant l'application.